# EUROPEAN PATENT SPECIFICATION

(11) **EP 1 699 460 B1**
(45) Date of publication and mention of the grant of the patent: **13.06.2012**
(21) Application number: 04806854.8
(22) Date of filing: 10.12.2004
(51) Int. Cl.: A61K 38/16, G01N 33/574, A61P 35/04, A61K 39/395, C07K 7/00

(54) **USE OF ENZYMATIC INHIBITORS OF H-PRUNE FOR THE PREVENTION AND TREATMENT OF THE METASTASES OF TUMOURS OVEREXPRESSING H-PRUNE**
VERWENDUNG VON ENZYMATISCHEN HEMMERN VON H-PRUNE ZUR PRÄVENTION UND BEHANDLUNG VON METASTASEN VON TUMOREN MIT ÜBEREXPRESSION VON H-PRUNE
UTILISATION D'INHIBITEURS ENZYMATIQUES DE H-PRUNE POUR LA PREVENTION ET LE TRAITEMENT DES METASTASES DE TUMEURS SUR-EXPRIMANT H-PRUNE

(30) Priority: 11.12.2003 IT RM20030572
(43) Date of publication of application: 13.09.2006
(73) Proprietor: Zollo, Massimo, 80145 Napoli (IT)
(72) Inventor: Zollo, Massimo, 80145 Napoli (IT)
(74) Representative: Iannone, Carlo Luigi
(86) International application number: PCT/IT2004/000689
(87) International publication number: WO 2005/056043

(56) References cited:
- US-B1- 6 486 300
- COLLIER G R ET AL: "INHIBITION OF LUNG METASTASIS FORMATION BY A RAT OSTEOGENIC SARCOMA SUBCLONE USING PYRIMIDO-PYRIMIDINE DERIVATIVES" AUSTRALIAN AND NEW ZEALAND JOURNAL OF MEDICINE, ROYAL AUSTRALASIAN COLLEGE OF PHYSICIANS, SYDNEY, AU, vol. 15, no. 1, SUPPL 1, February 1985 (1985-02), page 127, XP008046052 ISSN: 0004-8291
- BANDO H ET AL: "EFFECTS OF ANTIPLATELET AGENTS ON PULMONARY METASTASES" GANN, JAPANESE CANCER ASSOCIATION, TOKYO, JP, vol. 75, no. 3, March 1984 (1984-03), pages 284-291, XP009013087 ISSN: 0016-450X
- BERTRAM J S ET AL: "INHIBITION OF NEOPLASTIC CELL GROWTH BY QUIESCENT CELLS IS MEDIATED BY SERUM CONCENTRATION AND CYCLIC AMP PHOSPHO DI ESTERASE INHIBITORS" JOURNAL OF CELLULAR BIOCHEMISTRY, vol. 18, no. 4, 1982, pages 515-538, XP002329792 ISSN: 0730-2312
- NI XIAOHUA ET AL: "Isolation and characterization of a novel human NM23-H1B gene, a different transcript of NM23-H1." JOURNAL OF HUMAN GENETICS, vol. 48, no. 2, February 2003 (2003-02), pages 96-100, XP002329793 ISSN: 1434-5161
- POSTEL EDITH H ET AL: "Mutational analysis of NM23-H2/NDP kinase identifies the structural domains critical to recognition of a c-myc regulatory element" PROCEEDINGS OF THE NATIONAL ACADEMY OF SCIENCES OF THE UNITED STATES OF AMERICA, vol. 93, no. 14, 1996, pages 6892-6897, XP002329794 ISSN: 0027-8424
- REYMOND ALEXANDRE ET AL: "Evidence for interaction between human PRUNE and nm23-H1 NDPKinase" ONCOGENE, vol. 18, no. 51, 2 December 1999 (1999-12-02), pages 7244-7252, XP002329795 ISSN: 0950-9232
- FORUS ANNE ET AL: "Amplification and overexpression of PRUNE in human sarcomas and breast carcinomas: A possible mechanism for altering the nm23-H1 activity" ONCOGENE, vol. 20, no. 47, 18 October 2001 (2001-10-18), pages 6881-6890, XP002329796 ISSN: 0950-9232
- ZOLLO M ET AL: "Prune and nm23-H1 and nm-23 H2 (NDP-Kinase) proteins: Involvement in cancer" AMERICAN JOURNAL OF HUMAN GENETICS, vol. 69, no. 4 Supplement, October 2001 (2001-10), page 273, XP009047948 & 51ST ANNUAL MEETING OF THE AMERICAN SOCIETY OF HUMAN GENETICS; SAN DIEGO, CALIFORNIA, USA; OCTOBER 12-16, 2001 ISSN: 0002-9297
- DATABASE Geneseq [Online] 26 June 2001 (2001-06-26), "Human cDNA clone (5'-primer) SEQ ID NO:5290." XP002329797 retrieved from EBI accession no. GSN:AAH08455 Database accession no. AAH08455 & EP 1 074 617 A (RESEARCH ASSOCIATION FORBIOTECHNOLOGY) 7 February 2001 (2001-02-07)
- DATABASE Geneseq [Online] 6 November 2003 (2003-11-06), "Human intracellular signalling molecule INTSIG-44, SEQ ID NO:44." XP002329798 retrieved from EBI accession no. GSN:ADA13362 Database accession no. ADA13362 & WO 03/031568 A (INCYTE GENOMICS, INC; YUE, HENRY; LU, DYUNG AINA, M; SWARNAKAR, ANITA;) 17 April 2003 (2003-04-17)
- DANGELO A ET AL: "The human cyclic nucleotides phosphodiesterase (PDE) Prune protein: A dual cellular compartment localization and functional properties." AMERICAN JOURNAL OF HUMAN GENETICS, vol. 71, no. 4 Supplement, October 2002 (2002-10), page 513, XP009047949 & 52ND ANNUAL MEETING OF THE AMERICAN SOCIETY OF HUMAN GENETICS; BALTIMORE, MD, USA; OCTOBER 15-19, 2002 ISSN: 0002-9297

## Description

The present invention relates to the use of enzymatic inhibitors of h-PRUNE for the prevention and treatment of the metastases of tumours overexpressing h-PRUNE, screening method for said inhibitors and diagnostic kit for the detection of the metastases thereof.

More particularly the invention relates to the use of enzymatic inhibitors of h-PRUNE for the prevention and treatment of the metastases of breast tumours, sarcomas, melanomas, neuroblastomas and screening method for said inhibitors thereof. Further the invention relates to a diagnostic kit for metastases of said tumours.

The human PRUNE (h-PRUNE) protein belongs to the DHH superfamily, which includes several phosphoesterases, such as the nuclear RecJ from bacteria and the pyrophosphatases from yeast and bacteria (Aravind et al., 1998).

The DHH superfamily can be divided in two main groups on the basis of a -C-terminal motif which is very well conserved within each group but not across the various groups. All the members of this superfamily possess four other motifs containing highly conserved charged amino acid residues predicted to be responsible of ionic bonds and to catalise the phosphoesterification. The main characteristic of these is the third motif, namely DHH (Asp-His-His), after which this superfamily was named.

RecJ protein is a DNA repair protein and, along with other nucleases and poorly characterised bacterial proteins, belongs to the first group; while PRUNE and polyphosphatases belong to the second group.

The gene PRUNE from *Drosophila* was originally characterised on the base of its mutant phenotype showing a brownish-purple colour of eyes due to the reduction of drosopterins, in contrast to the bright red eye of the wild-type fly (Timmons et al., 1996). While homozygous PRUNE mutants are viable and fertile, they are lethal by developing pseudo-melanotic tumours in the presence of at least a single mutation copy which interferes with the correct formation of imagine disc which will result in the wing (awd/K-pn; also named Killer of PRUNE).

Humans encode up to eight orthologs of *awd* gene (nm23), at least four of which encode for active nucleoside diphosphate kinases (NDKPs) which catalyse the phosphoryl group transfer from a nucleoside triphosphate to a nucleoside diphosphate (Lombardi et al., 2000).

Several tumours and highly proliferative cells overexpress nm23-H1 mRNA and the protein thereof and in most cases this overexpression is linked to early stages of cancer. Breast cancer is a complex disease which presents a difficult clinical management due to its biological heterogeneity and its wide spectrum of responsiveness to treatment (Keen et al., 2003). Improved knowledge of molecular mechanisms underlying tumourigenesis allowed the identification of an increasing number of biomarkers which have been correlated with various cancer prognosis at different steps of pathology development, thus resulting in the choice of the most suitable therapeutic treatment (Keen et al., Domchek et al., 2002).

Among well-established prognostic factors currently used in cases of primary breast cancer there are lymph node involvement, histological examination, tumour size, estrogen and progesterone receptor status, proliferation index, nuclear or histological grade (Kuru et al., 2003, Morabito et al., 2003). It would be useful to provide new molecules involved in subsequent transformation, invasion and metastases processes to be used as markers for the prognosis of the subsequent steps in the tumour pathology development.

In breast cancer and melanomas high expression of human nm23-H1 is associated with a decreased metastatic potential (Florenes et al., 1992). For the specific case of breast cancer the spread of metastases is responsible for virtually all cancer deaths.

To become invasive tumour cells need to change their adhesive properties, loose contact with other cells in the primary tumour and establish new contacts with the extracellular matrix of the host cells of tissues they invade. Within this context the modulation of protease activity surrounding the tumour cells, plays a critical role. To migrate from the primary tumour and exit the circulatory system for colonising secondary organs, tumour cells also need to gain motility functions.

To date several metastases suppressing genes have been isolated and characterised (Steeg et al., 2003). Within this group nm23 is known to induce a decrease of cell motility when it is overexpressed in tumour breast cells (Freije et al., 1997, Hartsough et al., 2001, Freije et al., 1997), influence anchorage-independent colonisation and induce the differentiation (Kantor et al., 1993; Leone et al., 1993; Howlett et al., 1994; Hartsough et al., 1998, Lombardi et al., 2000). In addition it has been demonstrated that an overexpression of nm23-H1 in the aggressive breast cancer cell line clone MDA-C100 significantly reduces its metastatic phenotype both *in vitro* and *in vivo* (Hartsough et al., 2000, Mao et al., 2001, Tseng et al., 2001).

The inventor in a previous study has demonstrated the interaction between h-PRUNE and nm23-H1 and the disruption of this interaction by nm23H1-S120G mutation (Reymond et al., 1999). Further it has been disclosed that amplification of several copies of h-PRUNE results in induction of cell proliferation and high levels of h-PRUNE expression, compared to the moderate or low nm23-H1 levels, are correlated to increased aggressiveness of sarcoma and breast carcinoma tumours (Forus et al. 2001).

Accordingly it is apparent the need to provide compounds suitable to inhibit the enzymatic activity of h-PRUNE to be used advantageously for the treatment and prevention of tumours and metastases thereof characterised by the overexpression of h-PRUNE.

Now the author of the present invention has identified a new enzymatic activity of h-PRUNE particularly correlated to metastases, particularly of breast cancer, and susceptible to therapeutic enzymatic inhibition. In fact, the author has now discovered that h-PRUNE possesses a cyclic nucleotide phosphodiesterase activity with a preferential affinity for cAMP over cGMP as substrates, which can be effectively suppressed by some phosphodiesterase inhibitors.

In addition, through the study carried out by the author of the present invention, it was found that h-PRUNE enzyme results to be overexpressed with concurrent decrease of nm-23 expression in metastatic tumours in several tissues. Further it was found a direct correlation between increased activity of cAMP-PDE h-PRUNE and cell motility, due to a physical protein-protein interaction with nm23H1, in a breast tumour model. This discovery underlines the interaction between h-PRUNE and nm23-H1 resulting in the modification of the protective function of nm23-H1 in the cell proliferation and suppression of the tumour metastasis processes.

According to a further aspect of the invention, the possible role of h-PRUNE as potential independent marker for prognosis of the clinical development of breast cancer was evaluated by studying the distribution of the expression of h-PRUNE protein and of nm23-H1 in a group of patients with breast carcinoma. Particularly, the h-PRUNE overexpression, distributed homogeneously among the various analysed clinical cases, offers the chance of an advantageous use of the protein as prognosis marker independently from other factors such as, for instance, tumour type, histological sizes, estrogen and progesterone receptors reactivity, lymph nodes involvement. The identification of a new marker is useful to identify the tumours with metastatic potential and make more effective the management of the patients afflicted by breast cancer, in terms of choice of targeted therapy to be adopted.

Among the phosphodiesterase (PDE) inhibitors which were tested through a suitable screening method for the evaluation of the inhibition of hPRUNE cyclic nucleotide phosphodiesterase activity, dipyridamole, a drug already known for its anticoagulant properties, has revealed the highest ability to inhibit the h-PRUNE activity in terms of IC₅₀, both *in vitro* and breast cellular model. Also vinpocetine and 3-isobutyl-1-methylxanthine (IBMX) in the screening shown promising values of IC₅₀ for the h-PRUNE inhibition; the data from this selection suggest to address the screening to structural analogous, derivatives and isomers of the above mentioned three compounds.

Finally, in vivo analysis of many metastatic breast tumours confirmed the existence of a direct correlation between increased levels of h-PRUNE protein and negative regulation of the nm23-H1 expression, which results in the distant metastasis formation as reported in the clinical follow-up study.

Therefore it is an object of the present invention a peptide comprising the following amino acidic sequence: NIIHGSDSVESAEKEGGGYGRKKRRQRRR (SEQ ID No 10) characterised in that it is permeable because of the sequence GGGYGRKKRRORRR.

In addition, the present invention concerns a peptide comprising the following amino acidic sequence: NIIHGSDSVESAEKE GGGYGRKKRRQRRR (SEQ ID No 10) and characterised in that it is permeable and it is an inhibitor of h-Prune cyclic nucleotide phosphodiesterase activity, for use in medical field.

It is further object of the present invention the use of the above peptide for the preparation of a medicament for prevention and treatment of tumour metastases characterised by an overexpression of h-PRUNE. For example, the tumours characterised by an overexpression of h-PRUNE cam be breast carcinoma, sarcoma, neuroblastoma, prostate tumour, pancreatic tumour, colon carcinoma tumour, rectal tumour, medulloblastoma, epitelioma, epatocarcinoma, cell T or cell B lymphomas, myeloma and melanoma, and pulmonary tumour.

Furthermore the present invention concerns a screening method for h-PRUNE-inhibiting compounds, comprising the following phases:
a) selection of at least a phosphoesterase (PDE) inhibiting compound or derivative, structural analogue or isomer thereof;
b) administration of said at least one compound at concentration between 0,05 µM and 10 µM in a cell line overexpressing h-PRUNE, wherein said cellular line is MDA-C100 435 prune #4;
c) quantitative analysis of the cyclic nucleotide phosphodiesterase activity of h-PRUNE and/or analysis of cellular motility versus concentration of said at least one compound and chemo-attractant and selection of compound able to inhibit said phosphodiesterase activity between the values from 0.01 to 1 pmol/min⁻¹/ug⁻¹ and/or inhibit said motility up to the attainment of the values between 200 and 1200 cells.

The inhibition of the cyclic nucleotide phosphodiesterase activity of h-PRUNE by a compound tested as inhibitor of said activity can be established by evaluation of IC₅₀ for the aforesaid compound.

The quantitative analysis of step c) can be carried out by hydrolysis tests of the c-AMP and/or c-GMP substrate. Said substrate is used at concentration between 0,008 µM and 1 µM.

It is a further object of the present invention a method for in vitro detection of h-PRUNE in a biological sample, such as tissue section or biological fluid, for metastases diagnosis of tumours characterised by an h-PRUNE overexpression by immunological assay comprising the following steps:
a) bring into contact said biological sample with the anti-h-PRUNE monoclonal antibody able to recognise and bind selectively the h-PRUNE recombinant protein, characterised in that it belongs to the IgM immunoglobulin class and is produced by 4G3/4 clone (deposited at the CBA in Genoa on 10/12/2004);
b) detection of the antigen-antibody complex;
c) quantitative analysis of the antigen-antibody complex.

Said anti-h-PRUNE antibody can be labelled with a radioisotope, fluorescent molecule or enzyme. The detection and quantitative analysis of the antigen-antibody complex can be performed by immunohistochemistry, immunoprecipitation, immunofluorescence, ELISA, immunoblotting analyses.

It is a further object of the present invention a diagnostic kit for the detection of h-PRUNE in a biological sample for metastases diagnosis of tumours characterised by an h-PRUNE overexpression comprising the anti-h-PRUNE monoclonal antibody that belongs to the IgM immunoglobulin class and is produced by 4G3/4 clone (deposited at the CBA in Genoa on 10/12/2004). For example, the tumours characterised by an h-PRUNE overexpression are breast carcinoma, sarcoma, neuroblastoma, melanoma. Said anti-h-PRUNE monoclonal antibody can be labelled with a radioisotope, fluorescent molecule or enzyme.

In addition, the present invention concerns a monoclonal murine antibody able to recognise and bind selectively the h-PRUNE recombinant protein, characterised in that it belongs to the IgM immunoglobulin class and is produced by 4G3/4 clone (deposited at the CBA in Genoa on 10/12/2004). This clone was obtained through the immunization of mice with the whole h-PRUNE recombinant protein in a fusion construct containing at the NH₂ terminal the maltose binding protein (pMAL5 construct). The protein was purified through a maltose affinity column chromatography and then injected in two mice, with 5 boosters, any one with 100 ug of purified protein.

The present invention now will be described by way of illustration, but not limitation, according to its preferred embodiments, with particular reference to the figures of attached drawings, in which:

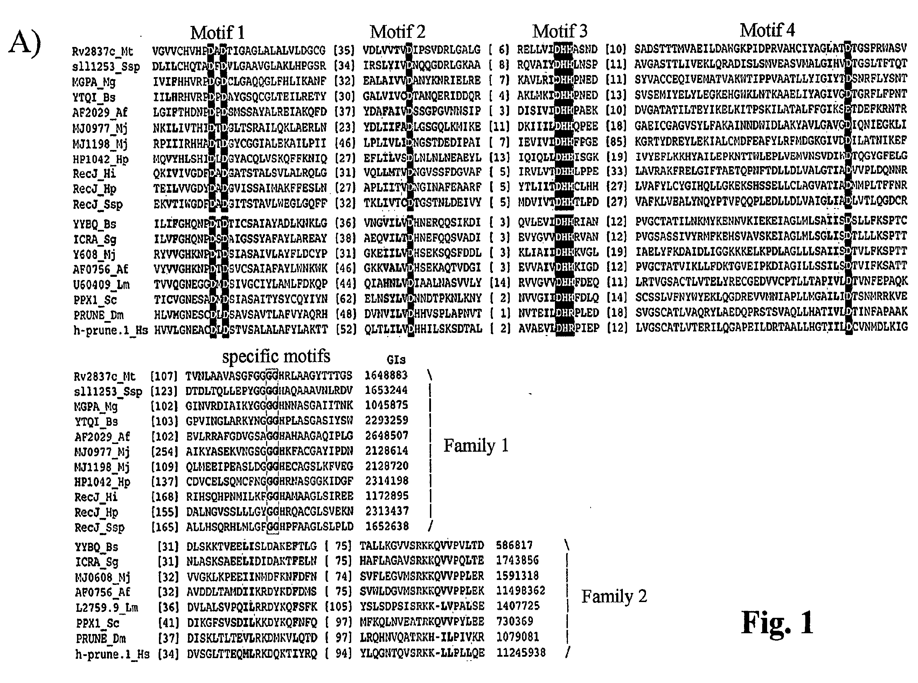

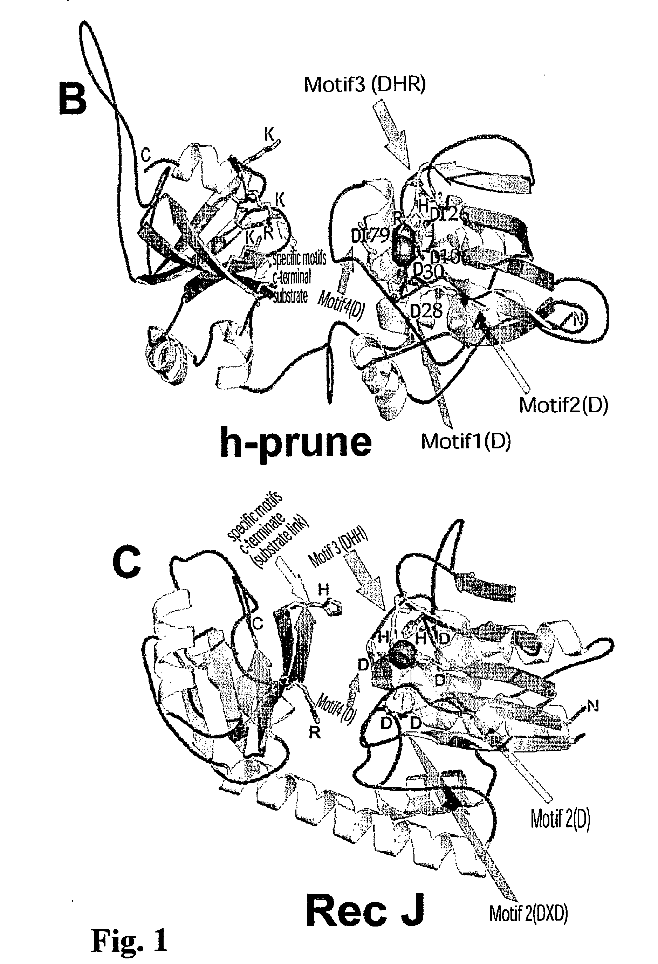

figure 1 shows multiple alignment of DHH family phosphoesterases sequences, showing separately the four generic motifs (I-IV) and the motifs diagnostic of the two distinct subfamilies mapping to the second domain; numerals indicate the positions of the first aligned residue in each protein sequence and distances between the different elements (panel A). "Ribbon" structure of the hPRUNE protein based on the crystal structure of PPASE and the RecJ protein (panel B) and "ribbon" structure of the RecJ protein (panel C); the arrows indicate aspartic acids residues (D);

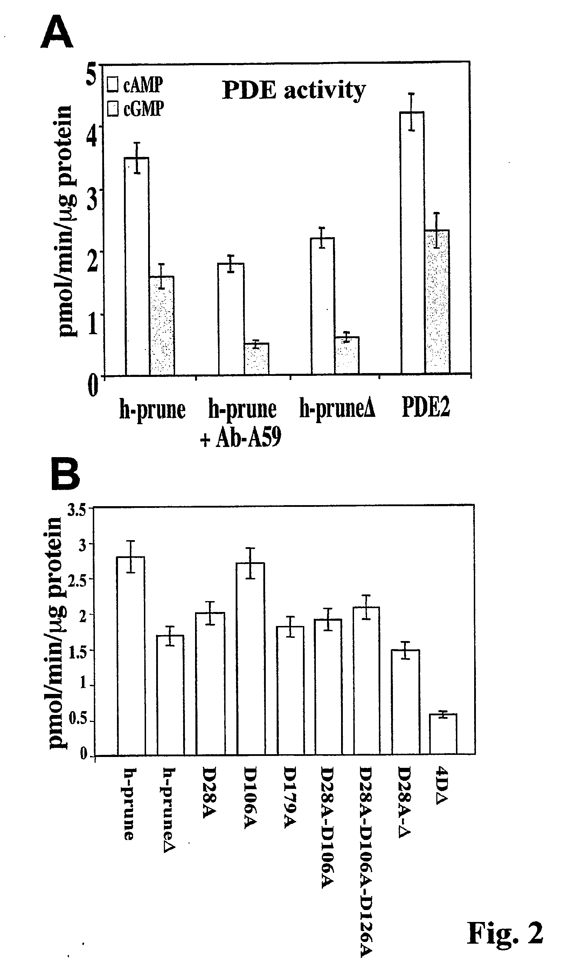

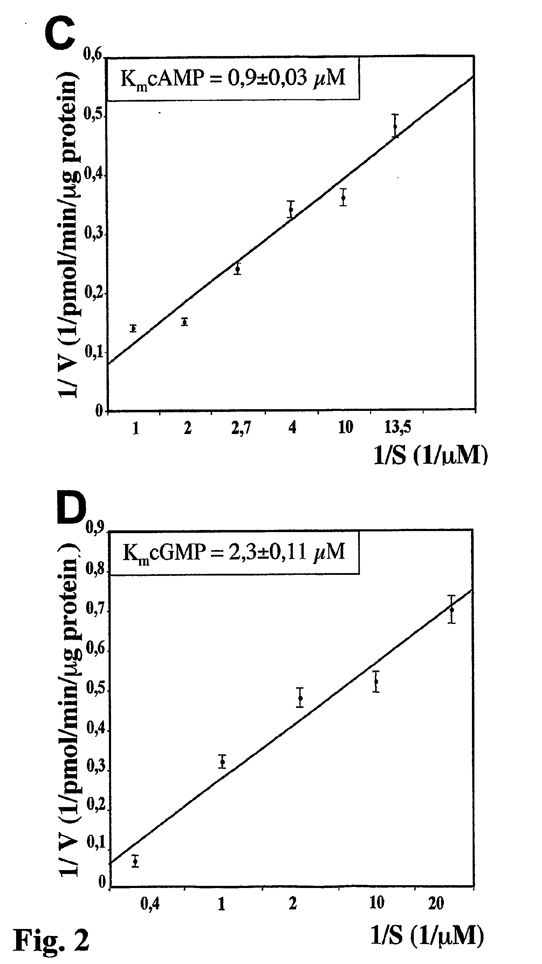

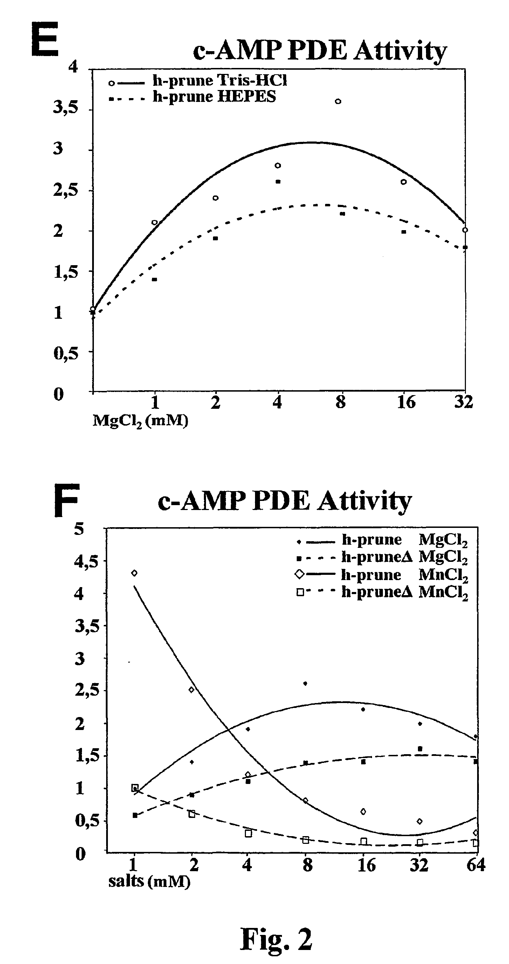

figure 2 shows the identification of h-PRUNE PDE activity on cAMP and cGMP substrates (panel A). Histogram of the analysis of single and multiple mutations mapping the potential catalytic site of h-PRUNE protein (panel B). Lineweaver-Burk plots to determine Kₘ and Vₘₐₓ for both cAMP and cGMP as substrate, respectively, (panels C, D). cAMP-PDE activity measured in the presence of two different buffers at increasing concentrations of Mg²⁺ (panel E). cAMP-PDE activity measured in the presence of increasing concentrations of Mg²⁺ (black points) or Mn²⁺ (white points). Activity plots of both h-PRUNE (solid lines) and h-PRUNEΔ (scattered lines) are shown (panel F);

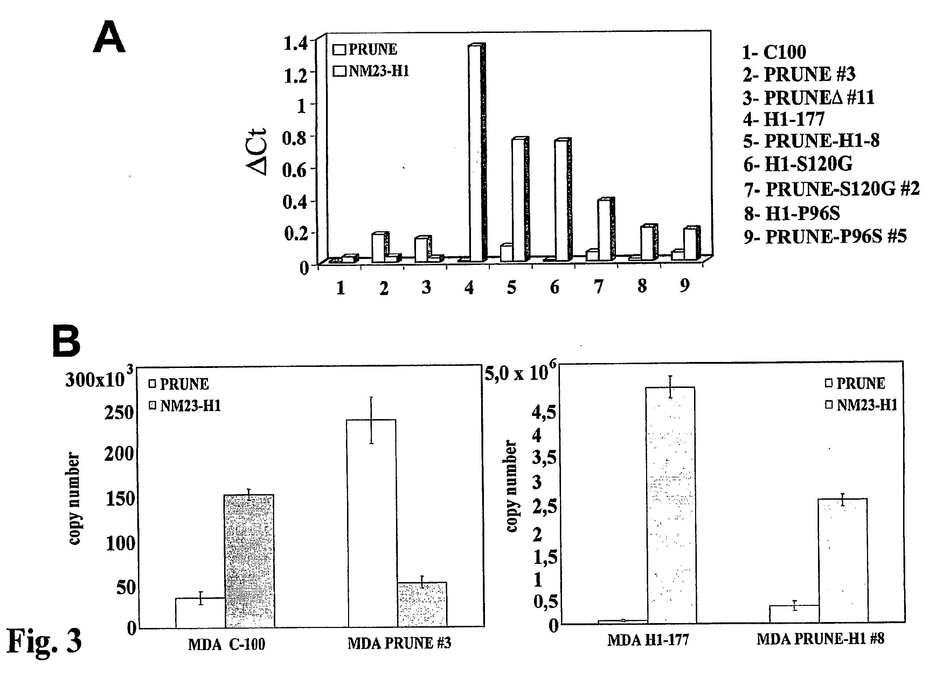

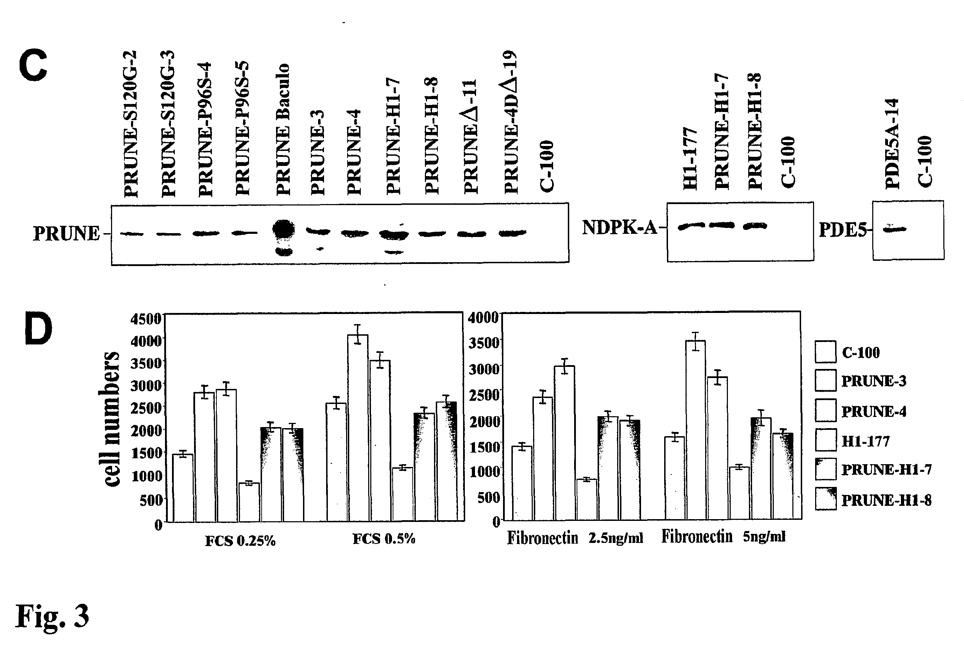

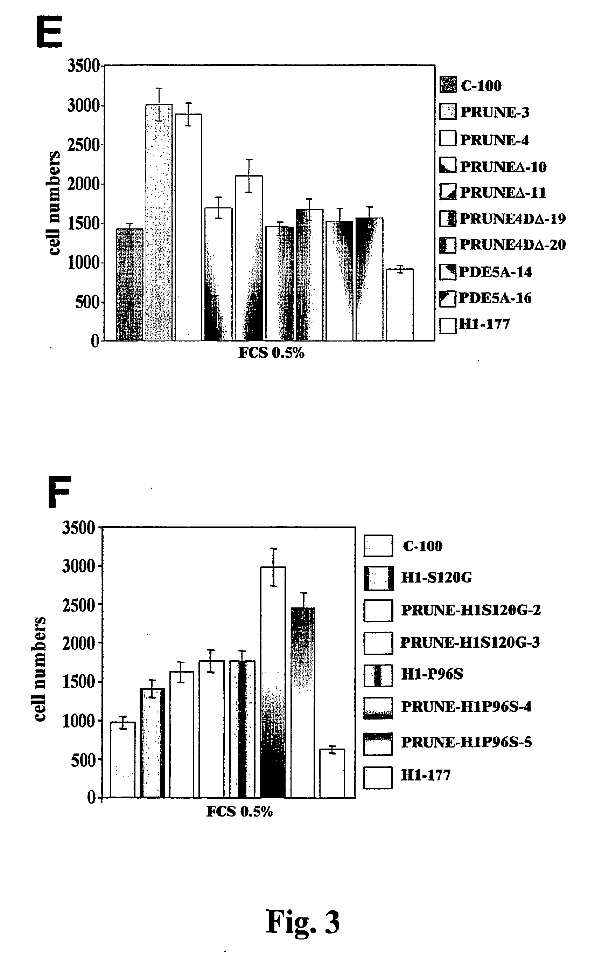

figure 3 shows stable clones analyses and *in vitro* motility assays. Examination of mRNA expression of h-PRUNE and nm23-H1 genes by Real Time PCR quantitative analysis; relevant ΔCt values are shown (panel A). Detection of the mRNA expression by Real Time; the copy number of m-RNA is reported (panel B). Western blot analyses using h-PRUNE, nm23-H1 and His-tag specific antibodies, respectively (for PDE5A) indicate the amount of proteins expressed in each individual cell clone (panel C). Cellular motility of MDA C-100 (control), MDA H 1-177, MDA-PRUNE and MDA-H1-177-PRUNE cell lines, overexpressing respectively h-PRUNE (clone #3 and #4) alone or h-PRUNE and nm23-H1 (clone #7 and #8), (panel D). Cellular motility of MDA C-100 (control), MDA-PRUNE (clone #3 and #4), MDA-PRUNEA (clone #10 and #11), MDA-PRUNE4DA (clone #19 and #20), MDA-PDE5A (clone #14 and #16) and MDA-H1-177 cell lines (panel E). Cellular motility of MDA C-100 (control), MDA-nm23H1-S120G, MDA-nm23H1-S120G-PRUNE (clone #2 and #3), MDA-nm23H1-P96S, MDA-nm23H1-P96S-PRUNE (clone #4 and #5) cell lines, overexpressing nm23-H1 mutants alone or with h-PRUNE (panel F);

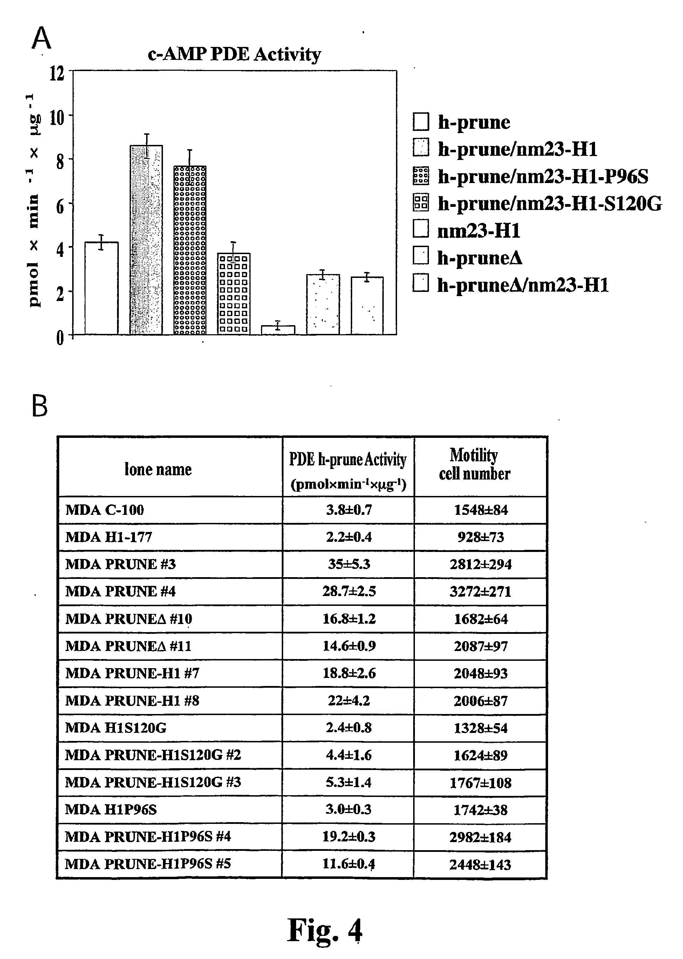

figure 4 shows the *in vitro* and *in vivo* h-PRUNE PDE activity analyses; h-PRUNE and h-PRUNEΔ cAMP-PDE activities in the presence of nm23 proteins (panel A). In the panel B table the values of h-PRUNE PDE activity measured as pmol x min-1 x µg⁻¹ on whole protein cell lysate are reported;

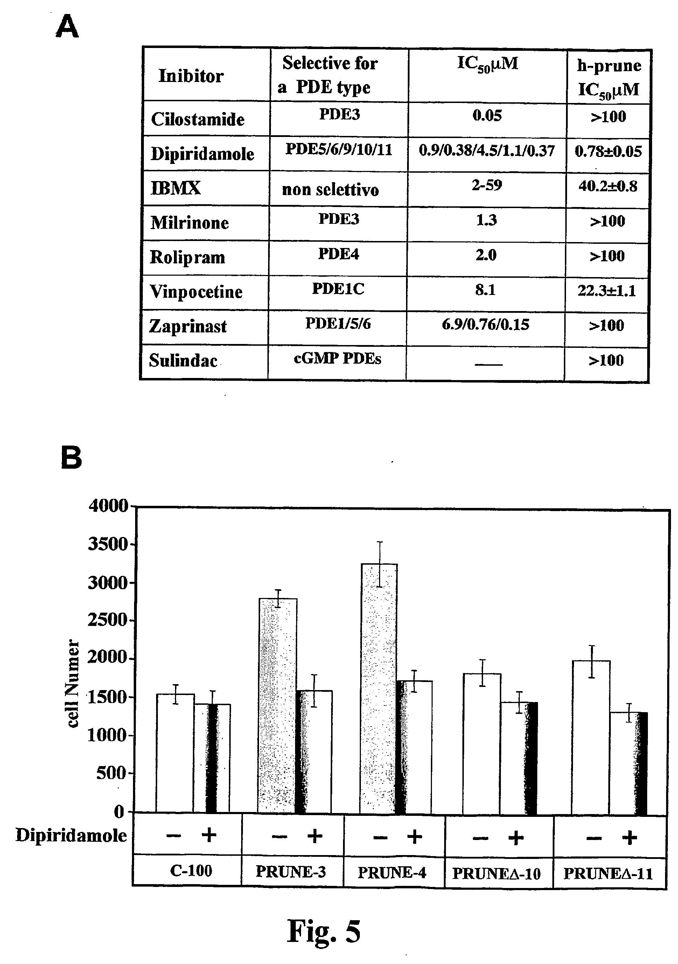

figure 5 shows the motility histogram representing analysis of the inhibiting activity on PDEs and motility of MDA C-100 (control), MDA-PRUNE (clone #3 and #4) and MDA PRUNEΔ (clone #10 and #11) cell lines (panel A). In the panel B table the inhibition activities on different PDE by 8 tested inhibitors, expressed as values of IC₅₀, are reported; in the last column h-PRUNE IC₅₀ values for some most sensitive compounds are shown.

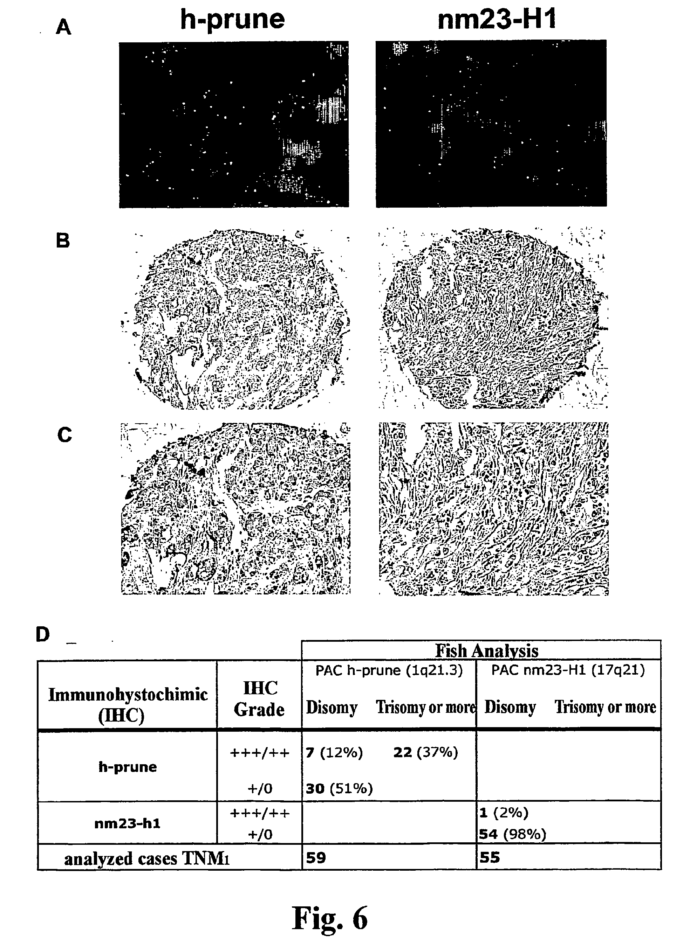

figure 6 shows *in vivo* analysis of breast tumour associated metastases; FISH analysis on MTA (multiple tissue array) that shows amplification of h-PRUNE (left) and nm23-H1 (right) (panel A). 100X and 200X magnification of immuno-histochemical analyses (IHC) of two groups of h-PRUNE highly expressing (+++) tumours (left) compared to moderate and low (0/+) nm23-HI expression level (right) (panels B, C); result table of both FISH and IHC analyses performed on 59 TNM₁ breast cancer cases (panel D).

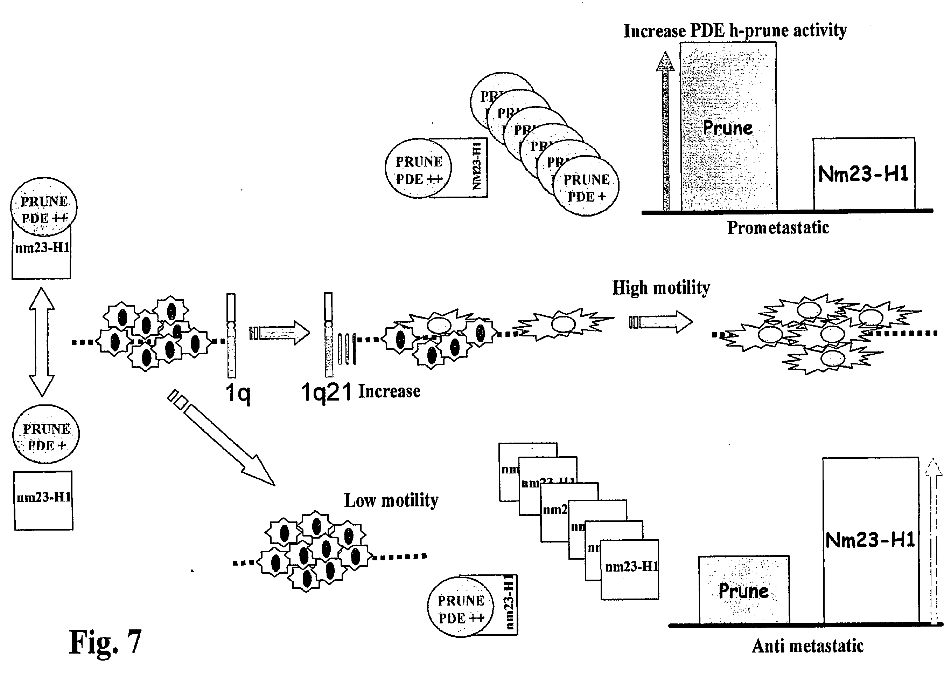

figure 7 shows a model representing h-PRUNE pro-metastatic function in breast cancer

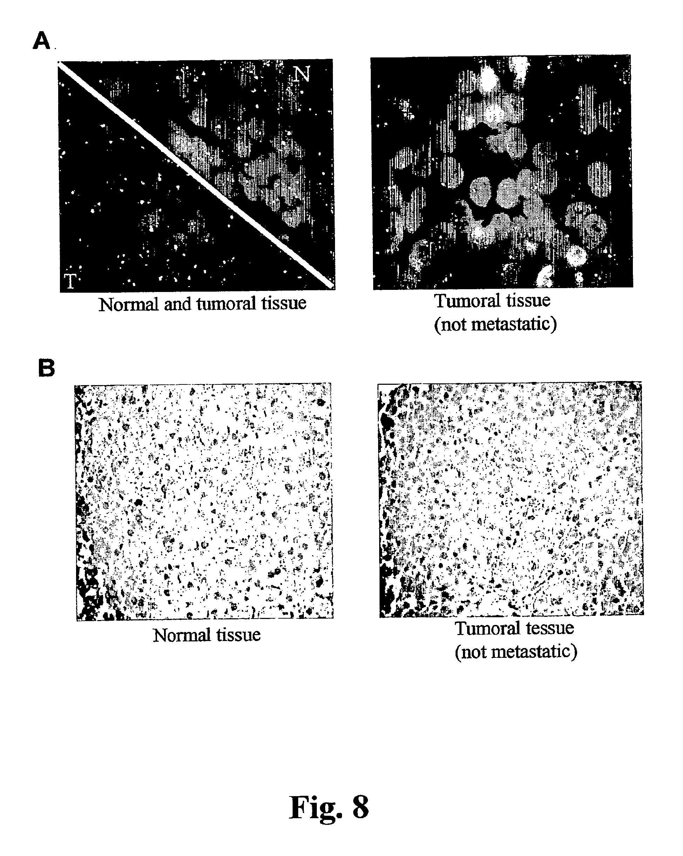

figure 8 shows FISH and immunohistochemistry analyses comparing normal and tumour breast tissues; in panel A FISH analysis on MTA that allows to individualise the h-PRUNE copy number both in normal and tumour tissues (left) and in tumour but no-metastatic tissues (right) is represented; in panel B magnification of the immuno-histochemical analysis performed on two groups of low h-PRUNE expressing (+) tumours is reported;

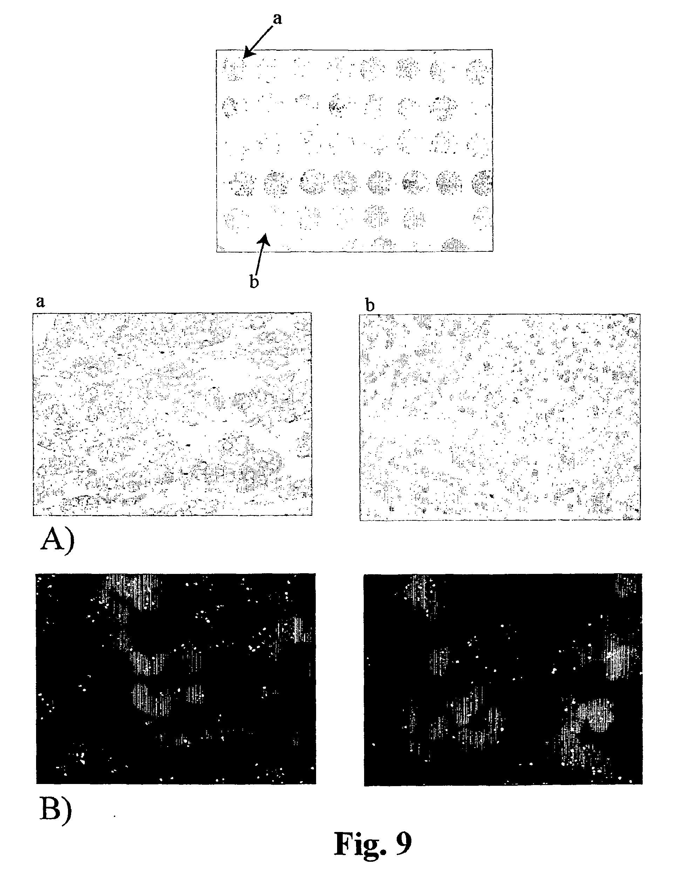

figure 9 shows expression and cytogenetic analysis of h-PRUNE in breast carcinoma. In the panel A the immunohistochemical analysis for h-PRUNE expression performed in TMA sections and 40X magnification of positive (a) and negative (b) h-PRUNE immunostaining are shown. In the panel B FISH analysis on the same samples employing h-PRUNE/PAC279-h19 as probes and pUC177 as control is reported;

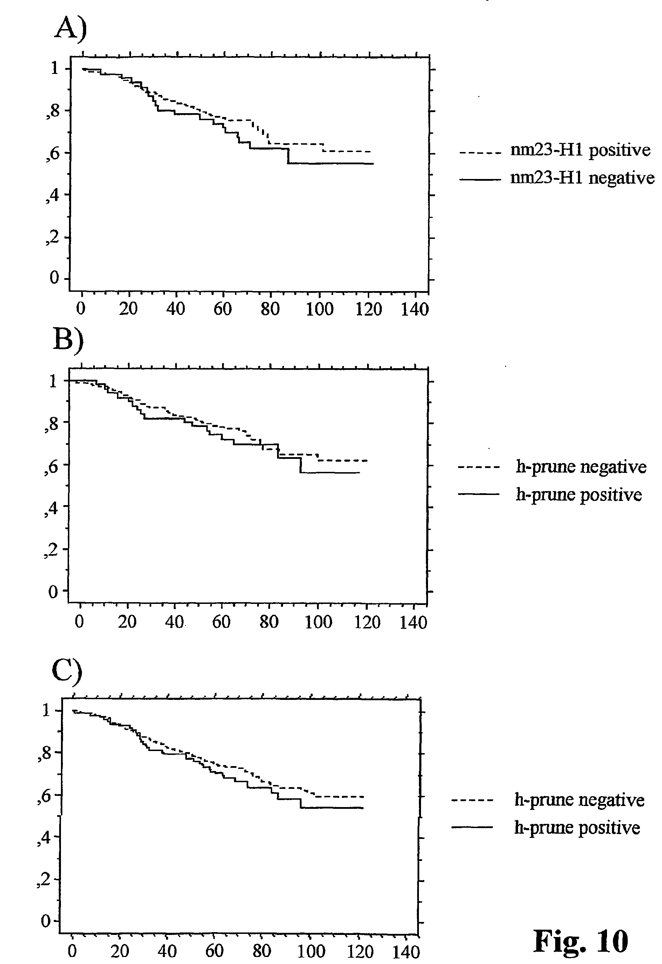

figure 10 shows Kaplan-Meier survival analyses on h-PRUNE and nm23-H1 expression. All cases of breast carcinomas were subjected to nm23-H 1 (A) and h-PRUNE (B) immunostaining; h-PRUNE-positive breast carcinomas in the presence (C) or absence (D) of axillary lymph node involvement;

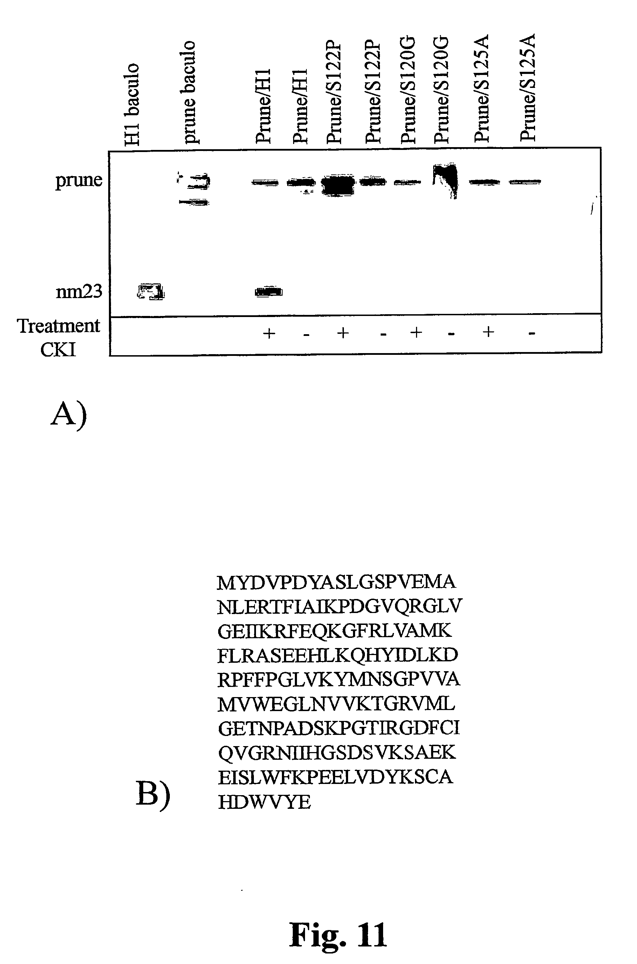

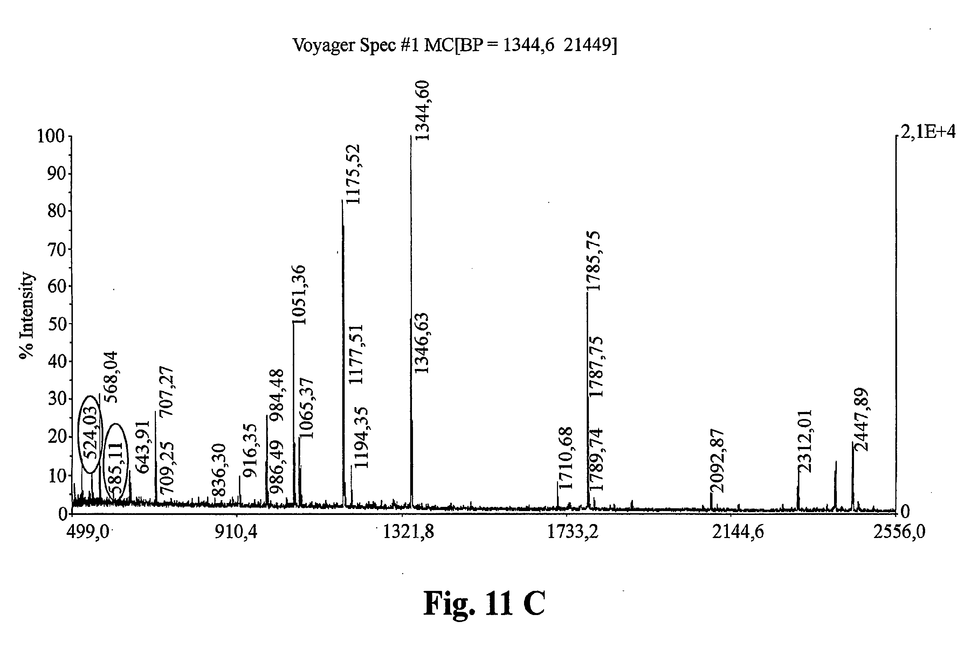

figure 11, A, shows *in vitro* immuno-precipitation of baculovirus produced h-PRUNE protein and Casein Kinasi δ *in vitro* phosphorylated and not phosphorylated nm23H1, several single mutations in S120-125 region were produced in order to show the complex formation through *in vitro* immuno-precipitation. The proteins are visible by Western blots using anti-HIS antibodies present in terminal NH2 of the recombinant proteins synthesised in Baculovirus. Lane 1 and 2 shows recombinant proteins as control. Immuno-precipitations were performed with A-59 anti-h-prune polyclonal antibody and the detection by W.B. with anti-His tag (prune/nm23) polyclonal antibodies; figure 11 B shows protein sequence and MALDI-MS of the protein, phosphorylation-positive serines are in bold type; figure 11 C shows COS7 expressed nm23-H2 using pcDNA-HA-nm23H2 construct and anti-HA recognising antibodies, following SDS page gel purification and trypsinization and sent to MALDI-MS molecular weight profile analysis, the identification of peptides and molecular mass thereof was carried out using Voyager mass-spectrometer with a mass increase corresponding to one or two phosphorylations. (80 Daltons, 160 Daltons);

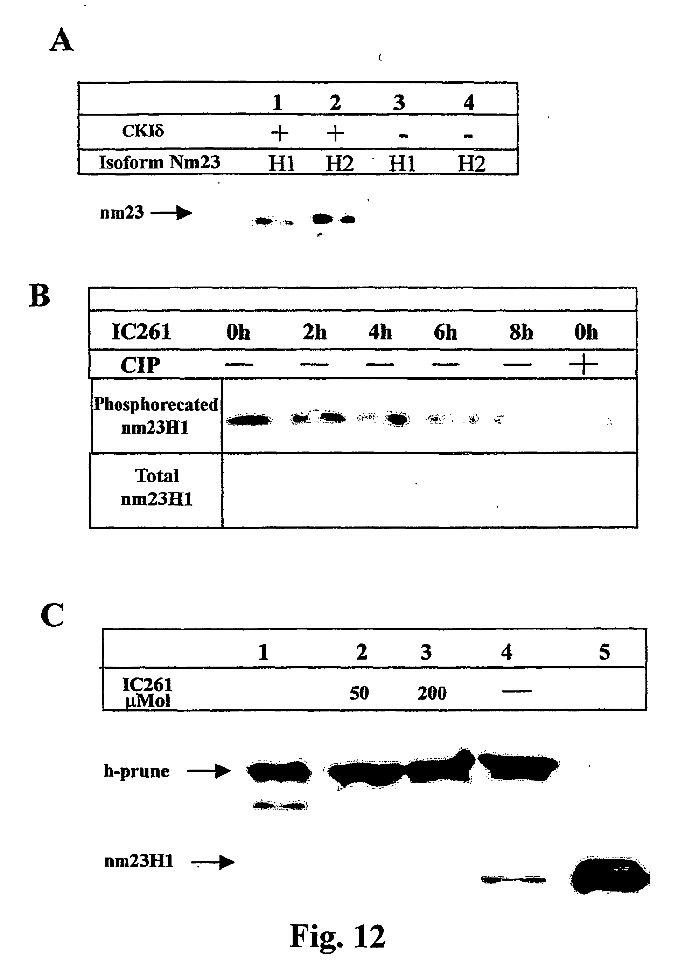

figure 12 A: K73 polyclonal antibody that recognises the peptide correspondent to nm23H1 phosphorylated region, obtained in rabbits, several times affinity purified against the phosphorylated peptide, lane 1 and lane 2 show that baculovirus produced nm23H1 and H2 recombinant protein, when *in vitro* phosphorylated by casein Kinase δ, is then recognised by the phosphorylated serine recognising IgG enriched specific K73 antibody. These data show the presence of specific phosphorylation in the Serine 120-125 region by a K73 specific antibody; figure 12 C: MDA H1177 were incubated with 50 mMol IC261 at different times, as described in example 4, 30mg of total extract were analysed for the phosphorylation of nm23 with K73 polyclonal antibody purified against the phosphorylated peptide. Lane 6 (given at 0 h) shows 30 mg of extract, used in lane 1, treated for 1 hour with 10U of ICP at 37°C. Fig. 12 D, MDA C100 were treated at two different concentrations with IC261 for 6h, h-prune was immuno-precipitated with A59 polyclonal antibody, the interaction with nm23-H1 was detected through moAb anti nm23H1 moAb (NOVOCASTRA). Lanes 1 and 5 show 20 ng of recombinant h-prune WB positive control and 20 ng of MDAH1177 cellular extract, respectively;

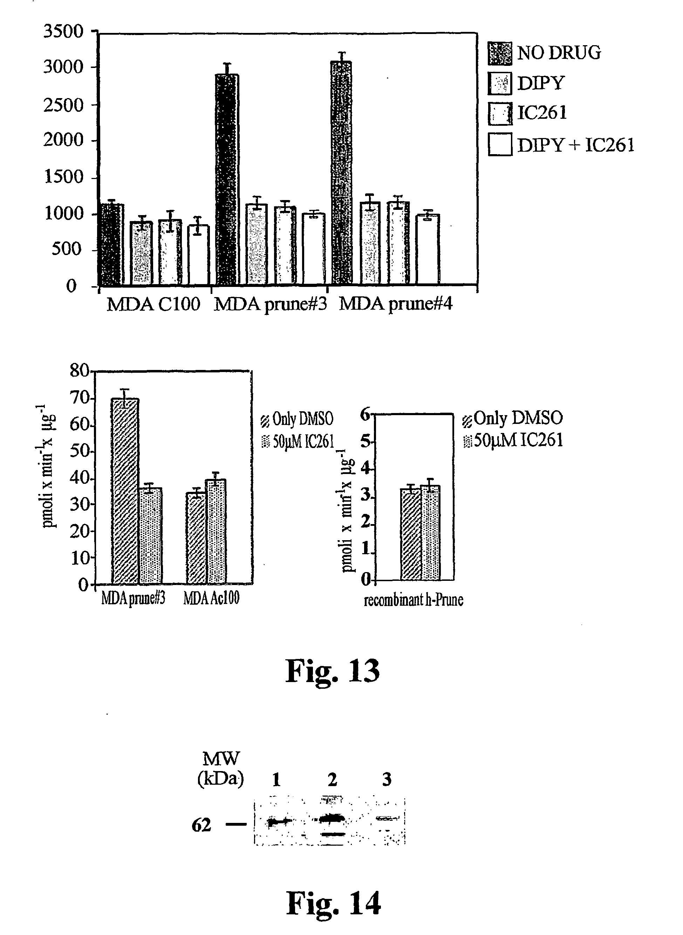

figure 13 A shows cellular motility values (arithmetic mean ± SD for five independent duplicate assays) following the treatment with dipyridamole, IC261 or dipyridamole and IC261; figure 13 B (lower left) shows that the presence of IC261 alters the content of linearised cAMP in MDA prune #4 cell (total content of cAMP-PDE activity) showing that the complex is responsible for the PDE-cAMP increase in the cells. In fact Fig.13 B (lower right) shows that recombinant h-prune protein *in vitro* in the presence of IC261 do not modify its biological activity, the specific activity value being similar to non-treated protein. From these data IC261 inhibits h-prune-nm23 complex, the latter being responsible for the *in vivo* increase of phosphodieterase activity, as it's apparent in MDA prune #4;
figure 14 shows h-prune detection by Western blot with a prune specific monoclonal antibody (4G3/4).

### EXAMPLE 1 : Structural and functional analysis of h-PRUNE

### MATERIALS AND METHODS

### Protein sequence analysis

The non-redundant database of protein sequences at the National Center for Biotechnology (NIH, Bethesda) was iteratively searched using the PSI-BLAST program (Altschul et al, 1997). Multiple alignments of protein sequences were constructed using the T_coffee program (Notredame et al., 1993) and corrected on the basis of PSI-BLAST program results.

Phylogenetic trees were constructed using the Philip Fitch program and Molphy package ProtML program.

Homology modelling of protein structures was performed by the SWISS-PDB-viewer software and the alignment with the template was manually adjusted to minimise the clashes of the protein backbone. The energy minimisation was carried out using the GROMOS program using a Sippl-like force field.

The diagrams of the ribbon structure proteins were generated using the MOLSCRIPT program.

### Cell culture

HEK-293 and MDA-MB-435 cells were cultured in Dulbecco's modified Eagles' medium supplemented **WITH 10 % FOETAL BOVINE SERUM, 100 UNITS/ml PENICILLIN**, and 100 µg/ml streptomycin at 37 °C with 5% CO₂.

### Protein expression and purification in Baculovirus

Protein expression was performed using Baculovirus Expression System (Invitrogen). In brief, the cDNA coding for h-PRUNE, nm23-H1 and the nm23-H1 (Mac Donald et al, 1996) and h-PRUNE mutants: h-PRUNEΔ, D28A, D106A, D179A, D28A-D106A, D28A-D106A-D126A, D28A-Δ, 4DΔ (D28A-D106A-Δ-D179A) were subcloned into an EcoRI/XhoI digested pFastBac-Hta vector.

To produce the h-PRUNE mutant cDNAs, site-directed mutagenesis of the h-PRUNE construct was carried out using the QuikChange III kit (Stratagene) according to the manufacturer instructions (see example 2).

Virus infection and purification conditions are described in Garzia and coil. (2003).

Subsequently histidine-tagged h-PRUNE and h-PRUNEΔ were purified on a MonoQ HR 5/5 column (Amersham) using 10 mM Tris-HCl pH 8.0 buffer. Column elution was performed with a linear gradient from 0 to 0.8 M NaCl, over 20 min and at a flow rate of 1 ml/min. The fractions were further dialysed against 10 mM Tris-HCl, pH 8.0 buffer, and tested for activity.

The purity of isolated proteins was measured by SDS page electrophoresis analysis.

### Identification and characterization of the h-PRUNE phosphodiesterase activity

PDE activity was measured by a cAMP/cGMP detection assay, as described by Fisher et al. 1998 and with a scintillation assay (Amersham-Pharmacia Biotech).

Samples were diluted and incubated at 30 °C in 100 µl assay buffer (50 mM Tris-HCl, pH 7.4, 8.3 mM MgCl₂, 1.7 mM EGTA) containing the desired concentration of cAMP or cGMP as substrates (unlabeled to ³H-labeled ratio = 3:1).

All the reactions, including blanks containing alone medium, were conducted in triplicate and allowed to proceed for an incubation time giving <25% substrates turnover (empirically determined).

Reactions were terminated by adding 50 µl Yttrium silicate SPA beads (Amersham).

Enzyme activities were calculated for the amount of radiolabeled product detected according to the manufacturer protocol. As negative controls h-PRUNE pre-incubated with A59 polyclonal antibodies, raised against the motif III region (Apotech Corporation, CH,) and h-PRUNEΔ mutant were used.

In particular, for PDE activity of h-PRUNE and h-PRUNE mutants 200 ng of purified enzymes were incubated for 10 min at 30 °C.

Lineweaver-Burk plots with Kₘ and Vₘₐₓ values were determined by measuring hydrolysis within a substrate concentration range (0.05-10.0 uM) and fixed amount of diluted enzyme over 5-40 min. Initial rates were calculated at each substrate concentration and plotted against the latter whereby the kinetic parameters were determined.

To study the influence of different buffers and nm23 activity on h-PRUNE PDE activity and perform inhibition studies, PDE assay was modified as reported in "materials and methods" section of example 2.

### In vitro cell motility assays

Stable MDA clones overexpressing h-PRUNE, h-PRUNEΔ, h-PRUNE4DA and human PDE5A were produced and analysed as described in "materials and methods" section of example 2.

Control breast cancer cell line MDA-C100 was used in the cell motility assay, as described previously (Leo et al., 1993) along with nm23-H1 overexpressing cell line (MDA-H1-177), which shows inhibition of metastasis processes *in vivo.*

Cellular motility was determined by means of the 6-well trans-well technology (Corning-Costar) using, as final concentrations, 0,25%, 0,5% FCS, 2,5 and 5,0 ng/ml fibronectin (Sigma), acting chemo-attractants, respectively (for further details see "materials and methods" section of example 2.).

### Statistical analysis

All the assays, including PDE activity and cellular motility ones, were validated using the T-test method available at http://www.graphpad.com/- quickcalcs/index.cfm.

FISH and HIC analyses, tumour clinical case collection and TNM selection are reported in "materials and methods" section of example 2.

### RESULTS

### Structural and functional analysis of h-PRUNE

An **ITERATIVE** protein data base search (PSI-BLAST) carried out on h-PRUNE protein, allowed to recover with statistically significant expectation values, the eukaryotic orthologs of PRUNE, followed by inorganic pyrophosphatases from a variety of bacteria and DHH proteins from various organisms, including the RecJ nucleases (figure 1, panel C).

Clustering of the DHH proteins using the BLASTCLUST program and phylogenetic analysis, using the maximum likelihood method, shows that PRUNE proteins (human and *Drosophila*) belong to the second DHH family, along with the inorganic pyrophosphatases (figure 1, panel A).

While the two families of DHH proteins share a common N-terminal domain that contains the four conserved motifs typical of the DHH superfamily, they are distinguished from each other by their C-terminal domains. Shared N-terminal domain has an α/β fold with parallel β-sheets and contains the absolutely conserved elements of the form DXD (Motif-I), D (Motif-II), DHH (Motif-III) and D/E (Motif-IV) (figure 1 panels A and B).

All of these residues are on the same face of this domain and together form the catalytic site chelating at least two divalent cations. PRUNE proteins contain the form DHR as substitution of the canonical DHH (Motif-III) that is observed in all other members of this family (figure 1, panel A).

In both DHH families, the C-terminal domain contains a core sheet of five strands, four of which form two β-strand hairpins. However, differences in the C-terminal domains between the first and the second family of DHH proteins may contribute prominently to substrate specificity of the two superfamilies. Additionally, C-terminal domain to the DHH module, mammalian PRUNE contains a non-globular extension within which there are some conserved serines that may play a role for regulation of the phosphorylation.

Structural analysis of h-PRUNE showed similarities to RecJ (Yamagata et al., 2002) and pyrophosphatases (Ahn et al., 2001), thus suggesting a potential similar activity to the latter.

However, their strong synergistic interactions with the awd/K-pn-like NDPKs suggested that PRUNE proteins might have alternative substrates, such as nucleotides.

Evolutionary studies about DHH family shown that phosphoesterases are derived from a number of protein folds containing various phosphoesterase and hydrolase domains. These include: the HD fold, from which the classic signalling PDEs are recognised (PDE1-11) (Aravind et al., 1998); the metal-β-lactamase fold (Galperin et al., 1999, Aravind et al., 1999), from which the PdsA-like PDEs are derived; and the calcineurin-like phosphoesterase fold (Aravind et al., 1998), from which Icc-like PDEs are derived.

The DHH catalytic domain has a very distinct fold from these other families and contains several analogous metal chelating residues (aspartic acid and histidines) that could potentially define an entirely new class of PDEs. In order to test this hypothesis, h-PRUNE was expressed, purified and assayed for potential PDE activity.

### Identification and characterisation of the h-PRUNE PDE activity

To determine the ability of h-PRUNE to hydrolyse phosphoester bonds in cyclic nucleotides (cAMP and cGMP), h-PRUNE was cloned and expressed using the Baculovirus expression system. His-tagged h-PRUNE and h-PRUNEΔ, a mutation created in the motif III region (DHRP126-129AAAA), were purified by affinity chromatography.

PDE assay was used to characterize purified h-PRUNE catalytic activity and to determine the specific substrate.

As shown in figure 2, panel A, h-PRUNE possesses significant PDE activity which is higher for cAMP than for cGMP substrate, while h-PRUNEΔ shows a 40% reduction of this activity. As positive control PDE2 was used; the negative controls were both h-PRUNE pre-incubated with A59 specific polyclonal antibody and h-PRUNEΔ.

To confirm the PDE detected activity, h-PRUNE and h-PRUNEΔ were overexpressed transiently in human HEK-293 cells and then immuno-precipitated and PDE assay was carried out on immuno-precipitated proteins.

These results indicate that h-PRUNE purified protein, both from insect and human cells, shows PDE activity.

To identify amino acids potentially involved in the catalytic site, a mutation analysis at single and multiple sites affecting h-PRUNE PDE activity was conducted. All the aspartic acid residues of the DHH characteristic motifs (figure 1, panel B) were mutated both alone and in combination (figure 2, panel B). The mutants were expressed using the Baculovirus expression system and purified to homogeneity (80% yield of purification).

The proteins were tested for their cAMP-PDE activity and it was observed an 80% decrease in the h-PRUNE 4DΔ (D28A, D106A, Δ, D179A) mutant activity. In summary, D28, D126, H127, R128, P129 and D179 amino acids were found to be essential for h-PRUNE PDE activity, thus indicating that most likely they are part of the catalytic site. Instead, D106A mutation in motif II did not influence h-PRUNE PDE activity.

To define Kₘ values, the His-tagged h-PRUNE protein was purified to homogeneity by another purification step using ion-exchange chromatography (Mono-Q column) with high purification yield (90%). Kₘ and Vₘₐₓ values were determined by measuring nucleotides hydrolysis with a fixed amount of purified enzyme within substrate concentration range (0.05-10.0 µM) and considering data points in the reaction linear part. Both cAMP and cGMP are substrates for h-PRUNE and show Kₘ values of 0.9±0.03 µM and 2.3±0.11 µM, respectively (figure 2, panels C and D).

Maximal rates of substrate turnover (Vₘₐₓ) were found to be 12.8±0.5 pmol x min⁻¹ x µg⁻¹ and 16.1±0.8 pmol x min⁻¹ x µg⁻¹ purified enzyme for cAMP and cGMP, respectively.

Thus evidence of a cyclic nucleotide phosphodiesterase activity for a protein of the DHH superfamily was pointed out. To study the buffer influence on h-PRUNE PDE activity, Tris-HCl and HEPES buffers were tested in the presence of same salt and higher PDE activity was observed in the presence of Tris-HCl buffer (figure 2, panel E).

Considering the ion dependence of DHH proteins, it was assessed the Mg²⁺ and Mn²⁺ ion dependency of h-PRUNE in the PDE cAMP assay. Although higher activity found of h-PRUNE in Tris-HCl buffer, PDE assays in the presence of HEPES buffer to avoid oxo-reduction reactions were performed and increasing concentrations of two different divalent ions were used.

Although some PDE activity was measured in the no-ion buffer, Mg²⁺ stimulated h-PRUNE PDE activity (figure 2, panel E); in contrast, in the presence of MnCl₂ this activity is inhibited. In addition, the h-PRUNEΔ mutant is not activated by the Mg²⁺ ions, as well as the wild-type protein (figure 2, panel F), thus indicating that the motif III, modified in h-PRUNEΔ mutant, is necessary for phosphodiesterase activity. In conclusion, it was shown that h-PRUNE cAMP-PDE activity is influenced positively by the Mg²⁺ ion concentration.

### Stable breast MDA h-PRUNE clones and correlation to cellular motility

To study the h-PRUNE function in regulating nm23-H1 anti-motility and metastasis suppressing activities, breast cancer MDA-C100 and HI-177 cellular models were employed (Hartsough et al., 2000, Mao et al., 2001, Tseng et al., 2001).

The MDA435-C100 cells (highly metastatic breast carcinoma cells, ATCC) were transfected with h-PRUNE gene cDNA in a LTR-containing plasmid construct (pBabe construct, which is employed to generate ecotropic retrovirus in mammalian cells), after transfection, different puromycin resistant clones were obtained (20 culture days to isolate clones), plasmid antibiotic resistance, and selected for h-prune cDNA overexpression thereof under the LTR promoter.

Several stable clones overexpressing h-PRUNE cDNA (clone #3 and #4, this last deposited at CBA in Genoa on 10/12/2004), h-PRUNEΔ cDNA (clone #10 and #11), h-PRUNE4DA cDNA (clone #19 and #20) and PDE5A cDNA (clone #14 and #16) in MDA-C100 cells were produced. h-PRUNE cDNA in MDA-H1-177 overexpressing nm23-H1 (clone #7 and #8), MDA overexpressing nm23H1-P96S (clone #4 and #5) and MDA overexpressing nm23H1-S120G (clone #2 and #3) was stabilised.

Some of these clones were characterised to determine the expression level of h-PRUNE mRNA using Real Time PCR analysis by TaqMan technology (figure 3, panel A). Four clones were selected for their level of mRNA expression by quantitative analysis and copy number extrapolation, to be compared to target reference gene (GAPDH) (figure 3, panel B).

In addition, Western blot analyses were performed to identify the expression levels of the h-PRUNE, nm23 and PDE5A proteins (figure 3, panel C).

Stable clones produced were then assayed for cellular motility using the Trans-well cell culture chambers (Freije et al, 1997, Hartsough et al., 2001, Freije et al., 1997). 6 independent clones (MDA-C-100; MDA-PRUNE clone #3 and #4, deposited at CBA in Genoa on 10/12/2004; MDA-H1-177-PRUNE clone #7 and #8; MDA-1I-177) were assayed. MDA-PRUNE clones have a 2-folds increase in motility when compared to the control cell line MDA-C100 (figure 3, panel D).

Values observed for the MDA-H1-177-PRUNE clone are increased of 2.2 folds as compared to the cell line MDA-H1-177, overexpressing nm23-H1 alone (figure 3, panel D). Clone MDA-H1-177 observed value is reduced by at least 40% compared to the MDA-C100 cell line, as previously reported (Hartsough et al, 2001), thus confirming the role of nm23-H1 for the inhibition of cellular motility.

In order to study the contribution of h-PRUNE PDE activity to cell motility, a motility assay on MDA-C100, MDA-H1-177, MDA-PRUNE (clone #3 and #4 deposited at CBA in Genoa on 10/12/2004), MDA-PRUNEΔ (clone #10 and #11) and MDA-PRUNE4DΔ (clone #19 and #20) was carried out. These mutants were chosen because of their different ability to influence h-PRUNE PDE activity.

A 40% and almost complete (90 %) decreases of cell motility in MDA-PRUNEΔ and in MDA-PRUNE4DΔ clones, both compared to MDA-PRUNE stable clones, were observed respectively. To verify whether h-PRUNE PDE activity contributes alone to cell motility in breast cancer cell lines, the clones overexpressing a well characterised PDE (PDE5A) in MDA-C100 (MDA-PDE5A clone #14 and #16) were tested. No increase in cell motility was observed in both PDE5A overexpressing clones (figure 3, panel E), thus indicating that only h-PRUNE PDE activity is able to induce cell motility in this conventional cellular model.

In addition, it has been reported (Freije et al., 1997, Hartsough et al., 2001, Freije et al., 1997) that the nm23H1-S120G (a mutant non-interacting with h-PRUNE) (Reymond et al., 1999) and nm23H1-P96S (a mutant retaining its ability to bind h-PRUNE) proteins are able to induce cellular motility.

The role of h-PRUNE in cellular motility activity overexpressing mutants with and without h-PRUNE was investigated and this was correlated to cellular motility. MDA-nm23H1-S120G-PRUNE clones show an almost 60% motility increase as compared to the MDA-C100 control cell line, while the MDA-nm23H1-P96S-PRUNE clones show a 200% motility increase when compared to MDA-C100 cells (figure 3, panel F).

In conclusion, the results indicate that overexpression of h-PRUNE in MDA-C100 cells increases cellular motility. h-PRUNE is able to revert the anti motility effect of nm23-H1, thus promoting cellular motility. This effect is not observed when h-PRUNE is overexpressed in the presence of h-PRUNE interaction impaired nm23H1-S120G mutant, thus postulating a role of nm23-H1-h-PRUNE complex on the increase of cellular motility.

### In vitro and in vivo h-PRUNE PDE activity

Considering that h-PRUNE and nm23-H1 physically interact (Mac Donald et al., 1996), it was examined whether nm23s may influence the PDE activity of h-PRUNE and the biochemical significance of the nm23-h-PRUNE interaction. This was achieved by pre-incubating nm23-H1 with h-PRUNE purified protein and measuring the cAMP-PDE activity *in vitro.*

h-PRUNE PDE activity showed up to a 2-fold increase over the control in the presence of nm23-H1. In addition, to verify that this increased activity is due to a physical interaction, different nm23 mutants were tested. The non-interacting mutant nm23H1-S120G (figure 4, panel A) was not able to increase h-PRUNE PDE activity; in contrast, the interacting mutant nm23H1-P96S increased h-PRUNE PDE activity almost as the wild-type nm23-H 1 , although to a lesser extent. This is possible because of the lower binding affinity to h-PRUNE, as previously reported (Reymond et al., 1999).

As a further control experiment, h-PRUNEΔ PDE activity was tested in the presence of nm23-H1 protein, because these two proteins do not interact during coimmuno-precipitation assays. There is no increase in h-PRUNEΔ PDE measured activity (figure 4, panel A).

These results demonstrate a correlation between the direct physical interaction of h-PRUNE and nm23-H1 and the increase of h-PRUNE PDE activity. Furthermore, each stable clone used in the motility assay was analysed for specific h-PRUNE cAMP-PDE activity on immuno-precipitated protein (figure 4, panel B).

As it result from these analyses, the MDA-PRUNE clones have 8 fold increase of cAMP-PDE activity as compared to the MDA-C100 one. Instead, the MDA-PRUNEΔ clones have a 0.5 fold decrease of cAMP-PDE activity as compared to the MDA-PRUNE and this correlates to their cell motility properties (figure 4, panel B).

In addition, it was found that h-PRUNE PDE activity in MDA-h-PRUNE clones compared to stable double MDA-H1-177-PRUNE ones is increased of 1.4-folds (figure 4, panel B).

These results show a direct correlation between h-PRUNE PDE activity and cell motility. Furthermore, MDA-nm23H1-S120G-PRUNE clones have a 3-folds decrease of h-PRUNE PDE activity as compared to the MDA-nm23H1-P96S-PRUNE ones, thus implying a direct correlation among h-PRUNE cAMP-PDE activity, cellular motility and protein-protein interactions. In conclusion, a direct correlation between h-PRUNE PDE function and protein-protein interactions, resulting in a significant influence on cellular motility, was pointed out.

### PDE inhibitor studies of h-PRUNE and influence on cellular motility

To identify the physiological role of h-PRUNE cAMP PDE activity in the cell a panel of selective PDE inhibitors was tested and it was verified whether any affects h-PRUNE protein activity.

The ability of h-PRUNE to hydrolyse cAMP was inhibited selectively by dipyridamole (already known to act against PDE5, PDE6, PDE9, PDE10 and PDE11). IC₅₀ measured for dipyridamole inhibition of h-PRUNE PDE activity was 0.78±0.05 µM and this value is lower (higher specificity) than other selective PDE inhibition values (PDE5, PDE9, PDE 10). Only PDE6 and PDE11 have a IC₅₀ value lower than h-PRUNE (figure 5, panel A).

h-PRUNE was also moderately sensitive to IBMX (3-isobutyl-1-methylxanthine) (IC₅₀; 40.2±0.8 µM), a non-selective specific PDE inhibitor, and vinpocetine (IC₅₀; 22.3±1.1 µM), a PDE1C specific inhibitor.

Several other inhibitors used in this study did not affect c-AMP h-PRUNE hydrolysis activity, even when applied at 100-fold higher concentration than for their IC₅₀ values against the other PDEs. Results of the inhibiting studies are summarised in figure 5, panel A.

Data presented above was further verified in MDA-MB-435 breast cancer cell line, in order to study the physiological inhibition *in vitro.*

It was chosen to use h-PRUNE overexpressing MDA clones and, as additional controls, MDA-PRUNEΔ ones, because of a partial reduction (40%) of h-PRUNE PDE activity as discussed above (figure 2, panels A and B), to verify at which extent dipyridamole was able to inhibit their activities and correlate them to cellular motility.

Both the MDA-PRUNE and PRUNEΔ clones were incubated with dipyridamole (8 µM, a 10-fold higher concentration than for IC₅₀) for 24 h to obtain the complete enzyme inactivation and then the motility assay was repeated as described above.

After treatment with dipyridamole, MDA-PRUNE and MDA-PRUNEΔ clones showed an average motility reduction of 40% and 20%, respectively, showing that the inhibitor acts against h-PRUNE PDE activity thus resulting in substantial decrease of cellular motility (figure 5, panel B).

### Breast carcinoma study on metastases affected patients

To verify *in vivo* the oncogenic role of h-PRUNE we have randomly selected fifty-nine cases for which metastases have been reported (TxNxMI according to TNM classification which describes the anatomical extent of disease (Hejna et al., 1999)).

Analysis was performed on a tissue multiple array (TMA) containing primary tumour tissues cases that showed metastasis at the time of diagnosis or during follow-up (at least 5 years of follow-up; date of diagnosis: 1992-97).

FISH analyses, using a PAC containing h-PRUNE (279-H19) as probe, revealed 22 out of 59 (37%) tumour cases with trisomy or higher copy number, indicating amplification of the h-PRUNE genomic region. Only one disomy and none amplification were observed in a total of 55 analysed cases using a PAC containing nm23-H1 as probe (figure 6, panel A).

In addition, immuno-histochemical analyses on TMA were performed using two antibodies (A59, K73) recognising h-PRUNE and nm23-H1, respectively. FISH and immunohistochemical analyses of normal as well as no metastatic cancer tissues were reported.

According to immuno-histopathology grading, in all of 22 cases (37%) after cytogenetic amplification of h-PRUNE chromosomal region it was also underlined high h-PRUNE protein expression in contrast to low or moderate expression level of nm23-H1, thus suggesting that about one third of breast metastasis formation may be due to both h-PRUNE amplification and overexpression with concurrent diminished level of the nm23-H1 metastases suppressing function.

In addition, 7 cases (12%) do not present h-PRUNE amplification but possess high h-PRUNE protein level while nm-23H1 level is low (figure 6, panel D). This suggests the presence of an alternative mechanism of h-PRUNE overexpression independent from gene amplification.

These data indicate a metastasis-promoting role of h-PRUNE protein in breast carcinoma.

### EXAMPLE 2: Gene expression profile of breast MDA stable clones

### MATERIALS AND METHODS

### Mutants preparation

The following oligonucleotides were used to generate h-PRUNE mutants:
h-PRUNEΔ (DHRP126-129AAAA): 5' - GTA GCA GAG GTG CTA GCC GCT GCAGCCATCGAG CCGAAACAC-3'(SEQ ID No 5);
D28A: 5'-GAA GCC TGT GCT TTG GAC TCC-3'(SEQ ID No 6);
D106A: 5'-ACC CTC ATC CTT GTC GCT CAT CAT ATC TTA TCC-3' (SEQ ID No 7);
D179a: 5'-GAA CCA TCA TCC TGG CAT GTG TCA ACA TGG-3'(SEQ ID No 8).

Mutated nucleotides are noted in boldface type and the altered codon is underlined. All mutations were confirmed by DNA sequencing.

Characterization of the h-PRUNE PDE phosphodiesterase activity

To study the buffer influence on h-PRUNE PDE activity the PDE assay was modified by using 50 mM Tris-HCl, pH 7.4 or 50 mM HEPES buffer, pH 7.5 at increasing concentrations (0, 1, 2, 4, 8, 16 and 32 mM) of MgCl₂.

To study the ion presence effect the PDE assays were performed in 50 mM HEPES buffer, pH 7.5 (in order to avoid oxo-reduction reactions) at increasing concentrations (1, 2, 4, 8, 16, 32 and 64 mM) of MgCl₂ or MnCl₂ salt. As a negative control, h-PRUNEΔ was used in the same conditions as for h-PRUNE. h-PRUNE activity in the absence of ions was tested after extensive dialysis of the protein against 50 mM Tris-HCl, pH 7.4, 1.7 mM EGTA or 50 HEPES buffer, pH 7.5, 1.7 mM EGTA.

The influence of nm23 (NDPK) activity on h-PRUNE PDE activity was investigated by carrying out the assays with a pre-incubation of purified h-PRUNE with various nm23 (70 % purification yield, -H1, -h1-S120G or -H 1-P96S).

The possible effect of nm23-H1 on h-PRUNEΔ was tested by pre-incubating the purified mutant protein with nm23-H1 and performing the PDE assay.

As to the inhibitor studies, eight different potential inhibitors were tested, namely: cilostamide, dipyridamole, 3-isobutyl-1-methylxanthine (IBMX), milrinone, rolipram, vinpocetine, sulindac and zaprinast (Sigma).

Low concentration of cAMP (0.01 µM) was employed in order to approximate IC₅₀ to Kᵢ. All inhibition studies were carried out in triplicate and were repeated thrice.

### Stable clones analyses

h-PRUNE, h-PRUNEΔ and h-PRUNE4DΔ cDNAs were subcloned into the EcoRI/Xhol digested pBABE vector modified with His-tagged at the N-terminus. Human PDE5A cDNA was PCR amplified by adding EcoRI and Xhol restriction ends in order to clone it into the same His-tagged vector.

The MDA-C100, MDA-H1-177, MDA-nm23H1-S120G and MDA-nm23H1-P96S clones were transfected with pBABE-h-PRUNE expression vector. MDA-C100 clone was transfected with pBABE-h-PRUNEΔ, pBABE-h-PRUNE4DΔ, and pBABE-h-PDE5A expression vectors.

Transfections were performed by using Lipofectamine (Invitrogen), according to the manufacturer instructions. Transfectants were selected in Dulbecco's modified Eagles' medium (DMEM) containing 10% foetal bovine serum, 100 Units/ml penicillin, 100 µg/ml streptomycin and 2 µg/ml puromycin (Sigma).

In order to prepare the bulk transfected lines, plates containing one hundred puromycin-resistant colonies were trypsinized, and cell lines were thus established.

Several puromycin-resistant pBABE-h-PRUNE, pBABE-h-PRUNEΔ, pBABE-h-PRUNE4DΔ and pBABE-PDE5A clones were isolated for every MDA clone, transfected and characterised both by Real time PCR and western blot analyses.

For real time PCR analysis, individual cell clones (MDA-C100, MDA-H1-177, MDA-H1S120G, MDA-H1P96S cell lines, several h-PRUNE- and h-PRUNEΔ stable clones) were plated at concentrations of 7-9 x 10⁶ cells in 75 cm² flasks.

mRNA was extracted and purified by TRIZOL® (Invitrogen) and then assayed by TaqMan quantitative Real Time PCR detection system using an ABI PRISM 7000 instrument (Applied Biosystems). Gene primer pairs were synthesised (Applied Biosystems: NME1-P/N 4331182; GAPDH) and Assay on Design was used to construct 6FAM probe sequence (6-carboxyfluorescein) both for the h-PRUNE and h-PRUNEΔ genes:
6FAM-CTGCATGGAACCATC (SEQ ID No 3);
FOR: AGAGATCTTGGACAGGCAAACT (SEQ ID No 1);
REV: CCATGTTGACACAGTCCAGGAT (SEQ ID No 2).

Several clones were selected and assayed for their mRNA expression levels by quantitative analysis and copy number extrapolation, as compared to reference target ones (GAPDH)

For western blot analysis, 15 µg of protein lysate in buffer were analysed by SDS-PAGE on 10 % (w/v) or 12.5 % (w/v) polyacrylamide gels and were electroblotted onto a PVDF membrane (Immobilon-P, Millipore). The lysates were immuno-detected with h-PRUNE specific polyclonal (A59, raised against the motif III region) for h-PRUNE, nm23-H1 (clone NM301, specific for the H1 isoform; Santa Cruz) for nm23-H1 and Penta-His against a His-tag (QIAGEN) for PDE5A antibodies, respectively.

After incubation with horseradish peroxidase-labelled anti-mouse IgG, visualisation was performed by enhanced chemiluminescence (Amersham). Eleven cell lines, namely MDA-PRUNE (clone #3 and #4), MDA-H1-177-PRUNE (clone #7 and #8), MDA-nm23H1-S120G-PRUNE (clone #2 and #3), MDA-nm23H1-P96S-PRUNE (clone #4 and #5) and MDA-PRUNEA (clone #11), MDA-PRUNE4DΔ (clone #19), MDA-PDE5A (clone #14), were selected and proteins were normalised on Western blots.

### In vitro cell motility assay

The trans-well technology was used (6-well - Coming-Costar). In the lower wells, 2.5 ml of DMEM containing 0,1% BSA, 100 Units/ml penicillin, 100 µg/ml streptomycin, 5 mM HEPES buffer (motility medium), and the diluted chemo-attractant were incubated; in the upper wells cells were incubated for 3 hours at 37 °C with 5%CO₂. After the attraction procedure, cells were fixed and stained with Gill n °1 hematoxylin solution according to manufacturer protocol (Coming-Costar); the cells were finally counted under the microscope.

### FISH analysis

PAC 279-H19 (PRUNE - chromosome 1q21.3) and PAC nm23-H1 (nm23H1 -chromosome 17q21) labelled by nick translation with dUTP-Cy3 (red) and control pUC177 labelled by nick translation with dUTP-FluorX (green) on 4 mm-thick formalin-fixed paraffin-embedded tissue sections (pUC177 was provided by Dr. M. Rocchi).

Nuclei were counterstained with 4',6-diamidino-2-phenyl-indole (DAPI). Two distinct experiments for each case were performed. Digital images were captured using an Olympus computerised epifluorescence microscope provided with COHU Video and Cytovision software. Hybridization signals on intact, well preserved, and non-overlapping nuclei were evaluated by at least two independent researchers.

### IHC analysis

MDA tumours were immuno-detected with specific A59 h-PRUNE polyclonal and nm23-H1 antibodies, respectively (clone K73, specific for the H1 and H2 isoforms; Apotech Corporation, CH).

Intensity of immunohistochemical staining was used to classify the tumour samples as positive if present in at least 20% of cells analysed under the microscope (strong +++, moderate ++, diffusely weak staining + or negative 0 (absent or focally weak staining) for h-PRUNE and nm23-H1 proteins expression.

### Tumour case collection and TNM selection

Tumour cases have been collected by AUSL1 of Sassari including patients with a minimum of 5 years follow-up. The TNM System classification was applied to this study as described by Sobin (Hejna et al., 1999) was used for describing the anatomical extent of disease and based on the assessment of three components: T corresponding to the extent of the primary tumour (from T0 to T4), N corresponding to absence or presence and extent of regional lymph node metastasis (from N0 to N4), and M standing for the absence or presence of distant metastasis (M0 or M1). The collection of 59 tumour breast cases was categorised as TxNxM1 positive.

### Gene expression in breast cellular clones

Probe preparation was carried out according to the following procedure.

RNA was extracted from MDA-H1-177-PRUNE (clone #8) and MDA-H1-177 clones by TRIZOL® method. Total RNA templates ranging from 15 to 20 ug were labelled using the Amersham CyScribe First Strand cDNA Labelling Kit with the following modifications.

Reverse transcription time was increased to three hours. The probe was then digested with Rnase-H for 30 minutes at 37 °C.

In order to eliminate the residual RNA, reaction mixtures were incubated with 0.25 M NaOH for 20 minutes and then neutralised with 2.8 M MOPS. The amount of incorporated Cy-dye was calculated for both Cy5 and Cy3. Labelled Cy3 and Cy5 targets were coupled in equal amounts and hybridised onto slides.

Probes with less of 50 pmoles of incorporated Cy-dye were not used for hybridisation.

Standard hybridisation protocol was applied to the slides. Two independent sets of experiments were performed.

### Scanning and Image analysis

Scanning and Image analysis were performed, respectively, by a Medway Far 2000-I and by Array Pro software from Media Cybernetics. Scanner sensitivity was kept between 900 and 1000 to compensate for both saturation and low intensity. Images in 16-bit format were processed.

### Data mining

In order to establish the statistical significance of any observed differences in genic expression between two experimental conditions each experiment was performed in triplicate.

In order to describe the data significance and variability a T-test was carried out on the several replicated experiments. Cyber-T tool available at the web page http://www.igb.uci.edu/servers/cybert was used to apply the test. A cut-off value at p=0.05 was used. As result of this analysis it is expected an almost of 5% false positives in different data sets. Only data sets that passed the test were used (data selection following T test).

### RESULTS

In order to verify the gene expression profile of the stable clones overexpressing nm23-H 1 alone and/or in combination with h-PRUNE, gene array studies were performed using a ready made array containing a 19,000 genes set. This array contains human Unigene cDNA data set spotted on glass slides (Ontario, Canada, Array facility, http://www.microarrays.ca/).

RNAs from MDA-H1-177 and MDA-H1-177-PRUNE (clone #8) clones were used to perform hybridisation. Two independent sets of experiments were performed on each breast cancer clone and T-test values (p=0.05) were extrapolated (http://www.tigem.it/zollo/cancercell/supplemental/array.html).

Expression levels of several genes involved in metastasis processes were found to be differentially regulated in the MDA-H1-177-PRUNE (clone #8) compared to MDA-H1-177 clone, as shown in Table 1. Table 1 summarises array experiments conducted on stable cellular breast clones; in the columns from left to right there are reported: the gene identification with ID number, Unigene reference number and position on human chromosomes; relationship between the array data of MDA-H1-177/MDA-H1-177-PRUNE clone #8 after two independent experiments conducted in duplicate; T-test value, including standard deviation and p value; bibliographical references of genes; processes of corresponding metastasis.

**Table 1**

| Gene Identification ID | Signal intensity ratio | Signal intensity ratio | | | | | | Gene references | Metastasis correlated processes |
|---|---|---|---|---|---|---|---|---|---|
| | Cy3/Cy5 | Cy5/Cy3 | | | | | | | |
| | (H1-177/Clone8) | (H1-177/Clone8) | #obs | Mn | SD | t | P | | |
| 310622:W31182:310622 :Hs.181385 : uncharacterized haematopoietic stem/progenitor cells protein MDS030:MDS030:9:9p24.1 | 1.441332 | 1.442391601 | 2 | 1.4418 | 0.0005 | 995,8991 | 0,006 | AUTHORS Zhao, M., Gu, J., Li, N., Peng. Y., Han, Z. and Chen. Z. TITLE Novel genes expressed in haematopoietic stem/progenitor cells from myelodysplastic syndromes patient JOURNAL Unpublished | Angiogenesis |
| 489392:AA054454:489392:Hs.821 59:proteasome (prosome, macropain) subunit, alpha type 1:PSMA1 :11 :11 p15.1 | 1.42474 | 1.423157331 | 2 | 1.4239 | 0.0007 | 635.9779 | 0.001 | Coux O;Tanaka, K; Golderberg, A; Structure and functions of the 20S and 26S proteasomes. Ann. Rev. Biochem. 65:801-847, 1996. | Protein Degrade I tion |
| 485121 :AA037777:485121:Hs.25300:phos phatidylinositol 4-kinase type II:P14KII:10:10q24 | 0.39234 | 0.394341204 | 2 | 0.3933 | 0.0035 | -366.7976 | 0.0017 | J Biol Chem 2001 May 18;276(20): 16635-40 Minogue S. Anderson JS. Waugh MG, dos Santos M. Corless S, Cramer R, Hsuan JJ. | Endocytosis and cytoskelethon rearrangiament |
| 4692752:BG538790:4692752:Hs.94 881 | 3.977598 | 4.013662507 | 2 | 3.9955 | 0.0063 | 306.9319 | 0.002 | Cancer Lett 1995 May 4;91(1):47-54 Sale-siotis AN, Wang CK, Wang CD, Burger A, Li H, Seth A | Metastasis |
| 307259:W21103:307259:Hs.84344: CGI-135 protein: LOC51024: 7:7q11.22 | 0.851047 | 0.857366017 | 2 | 0.8542 | 0.0052 | -42.6056 | 0.0149 | Hs. 38738 Homo sapiens CLDN15 Claudin 15 : Colegio OR, Van Itallie CM, McCrea HJ, Rahner C, Anderson -IM. Claudins create charge-selective channels in the paracellular pathway between epithelial cells. Am J Physiol Cell Physiol. 2002 Jul;283{1):C142-7. PMID: 120550B2 | Adhesion |
| 4862951:8076631 6:4862951 :Hs.347991:nuclea r receptor sub-family 2, group F, member 2: NR2F2:15:15q26 | 0.868882 | 0.862911048 | 2 | 0.8658 | 0.0048 | -41.7638 | 0.0152 | Pereira et al. (1999) . | Angiogenesis and development |
| 266005:N31521:266005:Hs.1986 89:bullous pemphigoid antigen 1, 230/2 40kDa :BPAG1:6: 6p12-p11 | 0.777256 | 0.787969285 | 2 | 0.7825 | 0.0096 | -35.8148 | 0.0177 | Brown, A.; Bemier, G.; Mathieu, M.; Rossant, J.; Kothary, R. The mouse dystonia musculorurn gene is a neural isoform of bullous pemphigoid antigen 1. Nature Genet. 10: 301-306, 1995 | Cytoskeleton organization |
| 278764:N98784:278764:Hs.148495: proteasome (prosome, macropain) 26S subunit, non-ATPase, 4: PSMD4:1 :1q21.1 | 0.695804 | 0.681432398 | 2 | 0.6885 | 0.0147 | -35.7552 | 0.0178 | .Deveraux, Q., Jensen. C.; Rechsteiner, M Molecular cloning and expression of a 26 S protease subunit enriched in dileucine repeats. J. Biot. Chem. 270:23726-23729, 1995. PubMed ID: 7559544 | Protein degradation |
| 54551 17:BM91 1128:54551 17: Hs.111779: secreted protein, acidic, cysteine-rich (osteonectin): SPARC:5:5q31.3-q32 | 1.472603 | 1.515475276 | 2 | 1.4938 | 0.0202 | 27.9731 | 0.0227 | Biochem Biophys Res Commun 2001 Sep 21;2B7(2):422-6 Induction of SPARO by VEGF in human vascular endothelial cells. Kato Y, Lewalle JM, Baba Y, Tsukuda M, SsKai N, Baba M, Kobayashi K, Koshika S, Nagashima Y, Frankenne F, Noel A. Foidart JM, Hata RI. | Angiogenesis |
| 321313:W321 80:321 313:Hs.387254 | 0.699574 | 0.671066627 | 2 | 0.6851 | 0.0294 | -18.1757 | 0.0349 | Sfiligoi C, De Luca A, Cascone I, Sorbello V, Fuso L, Panzone R, Biglia N, Audero E, Arisio R, Su ss olmo F, Sismondi P, De Bortoli M. Related Articles. Links Angio-poielin-2 expression In breast cancer correlates with lymph node invasion and short survival. Int J Cancer. 2003 Feb 1;103{4):466-74. | Angiogenesis |
| 320701 :W32046: 320701 :Hs.5415 | 0.772313 | 0.74904291 | 2 | 0.7605 | 0.0216 | -17.8901 | 0.0355 | Sfiligoi C, De Luca A, Cascone t, Sorbello V, Fuso L, Ponzone R, Biglia N, Audero E, Arisio R, Bussotino F. Sismondi P, DeBortoli M. Related Artiles, Links Anglopoletin-2 expression in breast cancer correlates with lymph node invasion and short survival. Int J Cancer. 2003 Feb 1;103(4):466-74. | Angiogenesis |
| 300555:W07526:300555:Hs.2936: matrix metalloproteinase 13 (collagenase 3): MMP13:11: 11q22.3 | 1.094073 | 1.105961019 | 2 | 1.1 | 0.0076 | 17.6383 | 0.036 | Del Casar Lizcano JM, Vizoso Pineiro F, Gonzalez Sanchez LO, Marlin Suarez A, Gava R, Cuesta Fernandez E, Diez San-tisteban MC. Relaled Articles, Links [Expression and clinical significance of collagenase-3 (MMP-13) in gastric cancer] Gastroenterol Hepatol. 2003 Jan;26(1):1-7. Spanish. COLLAGENASE 3 PRECURSOR | Metastasis |
| 429533:AA011457:429533;Hs.2376 17: dipeptidyipeptidase 9: DPP9:19: 19p13.3 | 1.185238 | 1.164378856 | 2 | 1.1 52 | 0.0131 | 17.3649 | 0.0366 | IV Olson C. Waghmann N, Identification and characterisation of human DPP9, a novel homologue of dipeptidyl peptidases IV Gene 2002 Oct. 18:2991-(1-2): 185-93 | Tumor biology (metastasis) |
| 4747535:BG676028:4747535:Hs.89 626: parathyroid hormone-like hormone: PTHLH: 12:12p12.1- | 0.944871 | 0.951010591 | 2 | 0.8479 | 0.0045 | -16.5107 | 0.0385 | Linferth R., Anderson N, Boey R, Nolan T., Downey S, Brady G, Ashcroft L, Bundred N, Coexpression of parathyroid hormone related protein and its receptor in early breast cancer predicts poor patient survival Clin Cancer Research 2002 Oct (10) 3172-7 PMID:12374885 | Metastasis |
| 504278:AA1 49501 :504278:Hs.332405:arginase,liver: ARG1 :6:6q23 | 1.391833 | 1.329405384 | 2 | 1.3602 | 0.0324 | 13.4093 | 0.0473 | Davel LE, Jasnis MA, de la Torre E, Goloh T, Diament M, Magenta G. Sacerdote de Lustig E, Sale ME. Arginine metabolic pathways involved in the modulation of tumor-induced angiogenesis of macrophages. FEBS Lett. 2002 Dec 4; 532(1-2); 216-20. | Angiogenesis |
| 127468:R08943:127468:Hs.269181 :ESTs,Similar to plakophilin 2 | 1 .34385 | 1.378604879 | 2 | 1.3611 | 0.0180 | 241,491 | 0.0263 | Plakophilins 2a and 2b: constitutive proteins of dual location in the karyoplasm and the desmosomal plaque. JOURNAL J. Cell Biol. 135 (4), 1009-1025 (1996) PUBMED 8922383MEDLINE97081101 | Adhesion |

Some genes, involved in oncogenesis and metastasis processes and significantly altered in gene expression analysis, were selected, and their expression level by an alternative method was confirmed.

Real time PCR experiments were performed on 11 genes (gene primers pairs were selected from GeneBank ID sequences). 7 and 4 out of these selected genes were found to be up- and down-regulated in MDA-H1-177-PRUNE clone (clone #8), respectively. Such gene expression differentiated at level of the stable h-PRUNE overexpressing clones, whose expression was confirmed both by chip-array and by PCR Real Time, is reported in table 2.

In the above described cellular models an up regulation of genes known to be involved in cytoskeleton re-organization (phosphatidylinositol 4-kinase type ii), protease activation (proteasome 26s subunit, nedd4 binding protein), oncogenesis (I-plastin, rab1b, braca1 associated protein (brap2), protein phosphorylation and nuclear transport (casein kinase ii 1-ckip1 interacting protein), was found.

While a down regulation was found in four genes, involved in extracellular matrix contacts or cell adhesion (Plakofilin, LIM), cytoskeleton re-organization (Plakin) and oncosuppressing activity (EXT1) and such evidences are reported in table 2. This table reports the different gene expression in stable h-PRUNE overexpressing MDA clones; in the columns from left to right there are reported: Genebank ID identification; gene name; relationship between array expression values of clone #8/clone #177 after two independent experiments conducted in duplicate; P value for array expression; relationship between the array expression values of clone #8/clone #177 valued by Real Time PCR; P value for Real Time PCR; correlated cellular biological process

**Table 2**

| UP-REGULATED | | | | | | |
|---|---|---|---|---|---|---|
| **Genebank ID** | **Gene Name** | **Array expression value ratio clone #8/clone 177** | **P value** | **Real Time expression value ratio clone #8/177** | **P value** | **Cellular biological precess** |
| NM 030981.1 | RAB 1 B ras family | 0.6902 | 0.0184 | 0.4005 | 0.0001 | Oncoqenesis |
| BT007330.1 | Phosphatidilynositol 4-kinase type II | 0.3933 | 0.0017 | 0.2230 | 0.0013 | Cytoskeletal rearranqiament |
| AF035620.1 | BRACA1 associated protein (BRAP2) | 0.7563 | 0.0027 | 0.0670 | 0.0062 | Oncogenesis |
| M22300.1 | PLASTIN | 0.7491 | 0.0078 | 0.0990 | 0.0001 | Oncoqenesis |
| NM_018177.2 | NEDD4 binding protein 2 (NABP2) | 0.7567 | 0.0145 | 0.2230 | 0.001 | Protease activation |
| NM_016274.3 | CK2 interacting preotein 1 (CKIP1) | 0.7929 | 0.0161 | 0.3499 | 0.0004 | Proliferation |
| XM_2080211 | 26-S PROTEASOME | 0.6886 | 0.0178 | 0.4475 | 0.0044 | Protease activation |
| R08943.1 | EST Ro8943 similar to plakophilin | 1.3611 | 0,0263 | 2.9180 | 0.4764 | Cell adhesion |
| AF061258.1 | LIM | 1.1717 | 0.0145 | 3.0525 | 0.0092 | Cell adhesion |
| BC0011741 | EXT1 | 2.1573 | 0.0398 | 2.9588 | 0.0623 | Tumor suppressor |
| NM_001723.2 | PLAKIN | 0.7826 | 0.0178 | 0.5625 | 0,0619 | Cytoskelethon organization |

In summary, genes involved in processes linked to oncogenesis were discovered to be significantly altered in their expression level, thus indicating a determinant contribution of h-PRUNE to the higher oncogenic potential of this breast cancer cell line.

### CONCLUSIONS

Structural analyses based on the two proteins belonging to the DHH superfamily, namely RecJ nuclease and inorganic pyrophosphatase, suggest that h-PRUNE is likely to possess a metal-ion-dependent phosphoesterase activity. As a result from DHH family structural analysis and evolutionary protein folding studies of phosphoesterases, it was demonstrated that mammalian h-PRUNE possesses a cyclic nucleotide phosphodiesterase activity.

Since h-PRUNE interacts with nm23-H1 isoform having NDPK activity, which is known to function on nucleotides, the attention was focused on the characterisation of the cyclic nucleotides phosphodiesterase activity and its role in cellular signalling. h-PRUNEΔ mutant containing substitutions in the conserved motif III of the DHH family shows a reduction of the PDE activity (figure 2, panels A, B, F).

Single or multiple mutation analyses at various sites indicate that only mutations in istidine (127), arginine (128), and proline (129) of motif III and aspartic acids D28, D126, D179, present respectively in motif I, III and IV, were found affecting substantially the h-PRUNE PDE activity, thus indicating most likely their involvement in the h-PRUNE PDE catalytic site.

The conserved motif III region is responsible for the binding of Mg²⁺ ions as predicted by protein modelling (figure 1, panel B), and, in addition, in an *in vitro* functional assay it was observed that h-PRUNE is also able to function in the absence (figure 2, panel E) or at low metal ion concentrations (figure 2, panel F).

It was demonstrated that h-PRUNE and the nm23s protein levels are unbalanced in sarcoma and breast carcinoma tumours, suggesting that h-PRUNE may negatively regulate nm23-H1 anti-metastatic function. An increase in h-PRUNE expression is directly correlated with aggressiveness of these tumours and cancer progression (Forus et al., 2001). Since several reports have postulated that the anti-metastatic activity of nm23-H1 is independent of the NDPK activity (Steeg et al, 1993, Wagner 1997), we investigated the h-PRUNE influence on cellular motility, which represents one of the first cellular functions to be acquired by the cancer cells to migrate away from the primary tumour site.

In order to confirm that h-PRUNE PDE activity is directly involved in these phenomena, h-PRUNE, h-PRUNEΔ and h-PRUNE4DΔ were overexpressed in a breast cancer model, and it was observed that overexpression of the wild type protein induces cell motility, while a decrease of its PDE function (h-PRUNEΔ, h-PRUNE4DΔ) corresponds to a decrease of cell motility.

On the contrary it was observed 80% PDE activity reduction for the mutant h-PRUNE4DΔ and that overexpression in MDA clone causes no significant increase in cell motility, thus excluding that other potential h-PRUNE activities are responsible of increasing motility.

Even though no other intracellular characterised PDE was associated to cell migration it has been investigated whether the increment of PDE activity in MDA stable clones might contribute to cell motility. To this end, PDE5A was overexpressed in the same cellular model and tested for influence on cell motility. PDE5A, chosen for its sensitivity to dipyridamole (IC₅₀ 0.9 µM), do not affect MDA breast cell motility, thus indicating that only h-PRUNE PDE function is responsible of increasing cell motility in breast cancer cells.

In addition, it has been observed that h-PRUNE overexpression in a high nm23-H1 expression background displays a decreased motility phenotype and lower h-PRUNE PDE activity compared to the cells overexpressing h-PRUNE alone. Although, h-PRUNE PDE activity is increased *in vitro* upon interaction with nm23-H1 (figure 4, panel A), this effect was not observed *in vivo.* These phenomena can be explained by the presence of a high amount of nm23-H1 in MDA-H1-177-PRUNE if compared to MDA-PRUNE clones (figure 3, panel B).

This can influence negatively the PRUNE-nm23H1 complex formation, which might depend on the presence of different oligomeric and/or post-translationally modified nm23-H1 forms (for example: serine phosphorylation) (Steeg et al., 2003).

In order to verify the hypothesis that the negative regulation of h-PRUNE on the nm23 anti-metastatic function is due to an increase in PDE activity as a result of the protein-protein interaction, it.was investigated the effect of two nm23-H1 mutants on h-PRUNE PDE activity. These protein mutants are nm23H1-P96S, able to physically interact with h-PRUNE, and nm23H1-S120G, that does not interact with h-PRUNE (Reymond et al., 1999); both mutants were transfected in breast cancer cells (MDA-435) and they are able to suppress the endogenous anti-motility effect of the nm23-H1 wild-type protein (Frije et al., 1997, Mac Donaldet al., 1996).

Additionally, it was shown that breast cancer cells overexpressing h-PRUNE in a high nm23 expression system, such as nm23-H1-S120G, have lower cellular motility in comparison to overexpressing h-PRUNE cells in a nm23-H1-P96S system.

Thus it was further demonstrated that the physical interaction between these two proteins may be responsible for the motility promotion. Furthermore, the h-PRUNE-nm23-H1-S120G clone has almost 66% lower PDE activity compared to the PDE value observed in the clone overexpressing both h-PRUNE and nm23-H1-P96S (figure 4, panel B), thus definitively indicating a correlation between protein-protein interaction, h-PRUNE cAMP-PDE activity and cellular motility effects.

In order to understand whether known PDE inhibitors were able to selectively function against h-PRUNE PDE activity, a set of 8 PDE inhibitors was tested and it was discovered that dipyridamole is able to inhibit h-PRUNE PDE activity with a significant IC₅₀ value.

In addition, the use of dipyridamole significantly reduces the motility of the stable h-PRUNE breast clones and at a lesser extent the h-PRUNEΔ overexpressing clones (figure 5, panel B). It is common opinion that anticoagulants such as dipyridamole and similar drugs exert their function interfering on the blood-clotting pathway activation through inhibition of adhesion of cells to capillary walls. In view of here reported results, it is reasoning to believe that the use of dipyridamole might represent a prevention and treatment drug for spread breast cancer metastases.

Moreover, the *in vitro* observed data on cellular motility activation were *in vivo* confirmed using a significant number of breast cancer tissues from TxNxM1 patients. In 59 tumours out of cases presenting distal metastasis, h-PRUNE was found amplified in copy number and overexpressed in 22 cases (37%), whereas nm23-H1 was found expressed at lower levels in all analysed cases (figure 6, panels B, C, D).

Preliminary data indicate a negative prognostic role of h-PRUNE in breast cancer as inferred by a statistically significant decrease of the overall survival rate among patients with overexpression of h-PRUNE. The presented data indicate that h-PRUNE up-regulates genes involved in metastasis and its activity *in vivo* increases the risk of more aggressive tumour behaviour, thus contributing negatively to the clinical outcome in breast cancer patients. Reported results have important pharmacological consequences, as the possibility to provide drugs able to selectively inhibit h-PRUNE PDE activity to be used in treatment of breast carcinoma in order to block h-PRUNE pro-metastasis malignancy function. Furthermore, 11 genes involved in oncogenesis and metastasis processes, whose expression was altered in breast MDA-H1-177-PRUNE (clone #8) if compared to MDA-H1-177 clone, were analysed by array expression and validated by Real Time PCR. The results presented are additional evidences of the aggressiveness and metastatic potential of the MDA breast cancer cell line correlated to h-PRUNE overexpression (example 2).

Overall, these results underline the role of h-PRUNE on promotion of cellular motility and metastases influencing negatively the nm23-H1 anti-metastatic function. The model employed for studying the role of h-PRUNE in cancer progression requires generally the amplification in tumour cells.

The amplification leads to an increased h-PRUNE PDE activity in cytoplasmic compartment, thus influencing negatively the suppressor of metastasis function of the nm23s. The activation of the h-PRUNE PDE activity is due to a physical interaction with the nm23-H1 protein; the complex formation results in a substantial decrease of free nm23-H1 form level, thus influencing cell proliferation, cellular motility and metastatic processes (figure 7).

EXAMPLE 3: *Study of the h-PRUNE overexpression role in diagnosis of invasive breast carcinoma.*

### MATERIALS AND METHODS

### Patients

In this study patients with a diagnosis of breast carcinoma confirmed by histological exam were included. Cases were retrieved from two archives of the University Hospital of Basel in Switzerland with the approval of the local Ethics Committee and of the Institute of Pathology at the University of Sassari, respectively. The first archive included 1,531 invasive ductal carcinomas, 310 invasive lobular carcinomas, 69 mucinous carcinomas, 65 tubular carcinomas, 48 medullary carcinomas, and 86 other types of invasive carcinomas. Formalin-fixed paraffin-embedded tumour samples were thus available from the Institute of Pathology, University Hospital Basel; the Institute for Clinical Pathology, Basel; and the Triemli Hospital, Zurich. The second archive included 307 invasive ductal carcinomas, 69 invasive lobular carcinomas, 12 mucinous carcinomas, and 24 other types of invasive carcinomas.

All of the clinico-pathological features for each patient, including disease stage at diagnosis, therapy, relapse, disease-free survival, and time of last control, were obtained from the registries of Basel and Sassari.

Pathological stage, tumour diameter and nodal status were obtained from the primary pathology reports. Clinical data were available after a 10-year follow-up for 2,299 cases (median, 56 months; range, 2-120 months); clinical data after a 15-year follow-up were available for 222 patients (median, 138 months; range, 121-176 months). The use of the specimens and data for this study was approved by the Ethics Committees at both Basel University Hospital and the University of Sassari.

### Tissue Multiple Array (TMA)

TMA construction was previously described (Simon et al. 2001; Simon et al., 2002). Summarising, tissue cylinders with a diameter of 0.6 mm were punched from representative tumour areas of a tissue donor using a semiautomatic robotic precision instrument and positioned in 6 different paraffin blocks, each containing between 342 and 522 individual samples. Four micrometric sections of the resulting TMA blocks were transferred to a coated section System (Instrumedics Inc., Hackensack, New Jersey). The presence of tumour tissue on the multiple-arrayed sample was verified by haematoxylin-eosin staining. Tissue microarrays included at least two sections from different areas of breast cancer from each patient as well as normal tissue as control.

### Immunohistochemistry (IHC)

Formalin-fixed, paraffin-embedded tissue sections were immuno-stained using anti-h-PRUNE specific monoclonal (clone 4G3/4, against a recombinant fusion protein from 1 to 351 amino acids; Apotech Corporation, CH) and anti-nm23-H1 (clone K73, -H1 isoform specific; Apotech Corporation, CH) antibodies. Immunohistochemical analysis was performed using the Vectastain Elite ABC Kit (vector Laboratories Inc.) according to the manufacturer instructions and following a known protocol (Forus et al., 2001; D'angelo et al., 2003).

Optimised IHC protocols were established by staining representative control histopathology sections of healthy breast tissue. In normal breast tissue, nm23-H1 protein was homogeneously expressed, whereas expression of h-PRUNE was absent or at low intensity.

Staining was evaluated semiquantitatively by using 54 normal samples randomly positioned in duplicate across the multiple arrays; intensity and distribution of immunohistochemical staining was used to classify tumour samples as positive (strong [+++] to moderate [++] staining, homogeneously distributed or presented by large majority of tumour cells) or negative (absent or weak staining [+]) for both h-PRUNE and nm23 expression.

### FISH analysis

Paraffin-embedded TMA sections were treated according to known protocols (Simon et al., 2002: Muresu et al., 2002).

The PAC 279-H19 clone, spanning the h-PRUNE gene region localised on chromosome 1q21.3, and DNA/BAC clone, specific for the nm23-H1 gene localised on chromosome 17q21.3, were labelled by nick translation with dUTP-CY3 (red) and used as probes. pUC177, corresponding to the peri-centromeric region on chromosome 1 q 12 and pZ17-14, corresponding to the centromeric region of chromosome 17, clones were labelled by nick translation with dCTP-FluorX (green) and used as controls.

Nuclei were counterstained with 4',6-diamidino-2-phenyl-indole (DAPI). Two distinct experiments were performed for each case.

Digital images were captured using an Olympus BX-61 epifluorescence microscope equipped with appropriate filters for excitation of DAPI, Cy3 (orange) or FluorX (Vysis), and a COHU video and Cytovision software. Hybridisation signals on at least 100 intact, well-preserved, and non-overlapping, nuclei were evaluated by at least two investigators.

### Statistical analysis

The following variables and categories were included in this study: pathological primary tumour size (pT), pathological nodal status (pN), presence of metastases (M), estrogen and progesterone receptor (ER and PR, respectively) status, age at diagnosis, histological tumour type, and overall survival (calculated starting from the time of surgical operation). In particular, nodal and receptor statuses were not available in a large number of the cases because these variables were not required for the inclusion of the patients in the study.

Survival data analysis was carried out with the statistical package Egret (version 2.0.3). The Cox regression model was performed using raw mortality and tumour-specific mortality incidence as censorship.

The time of overall survival was expressed in months and independent variables (h-PRUNE, nm23-H1, pT, pN) were stratified in three age groups: 23-44 (N=252), 45-64 (N=1,146), and 65-98 (N=1123).

Kaplan Meier estimates were executed by subdivision of h-PRUNE and nm23-H1 immunohistochemical data.

Using the Pearson's Chi-Squared test, h-PRUNE and nm23-HI expression assessing was performed in association with the pathological parameters (histological tumour type, pT, pN, M, ER, PR). The exact coefficient for sample proportion analysis was calculated to determine all of the significant parameters (below the 0.05 level). All analyses were performed with the statistical package SPSS/7.5 for Windows.

### RESULTS

Assessment of h-PRUNE and nm23-H1 expression levels, as well as h-PRUNE chromosomal copy number, was carried out on TMA sections from the archival tissues of 2,109 patients with a histologically proven breast carcinoma diagnosis and available follow-up data. Additional TMA sections from 412 breast cancer patients (i.e., whose TNM classification was available) were evaluated for h-prune immunostaining. The majority of patients had ductal carcinoma as the histological variant (1,883; 73%) and were >60 years of age (1,425; 57%) at the time of diagnosis. Data of the clinical follow-ups for each patient was available, covering a median period of 59 months (range 2-176); the majority of the patients were still alive (1,716; 68%), and only a very few cases were lost in the follow-up (12; 0.5%) at the time of this study. Expression of h-PRUNE and nm23-H1 proteins was evaluated by immunohistochemical analysis using two specific antibodies (4G3/4 and K73 monoclonal Ab clone, respectively). Figure 9 (panels A and B) shows representative examples of staining for h-PRUNE protein in a series of multiple tissue arrays.

A strongly positive cytoplasmic immunostaining for h-PRUNE protein (referred to as h-PRUNE+) was observed in the majority (1,340; 54%) of the 2,463 tested tumours (58 cases were not assessed); a positive cytoplasmic immunostaining for nm23-H1 protein was observed in 615 (30%) of the 2,061 tested tumours. Among the 2,061 tumour tissues evaluated for h-PRUNE and nm23-H1 expression, an inverse distribution of positive immunostaining was observed [1,180 (57%), breast carcinomas were h-PRUNE+, whereas 615 (30%) were nm23-H1+]. In table 3 are reported the immunohistochemical analysis (IHC) results for the h-PRUNE and nm23-H1 proteins present in invasive breast carcinomas. No statistical correlation between h-PRUNE and nm23-HI expression was observed.

**Table 3**

| IHC Analysis | nm23-H1 | | Total | |
|---|---|---|---|---|
| | Positive | negative | | P |
| Positive h-PRUNE | 359 (30%) | 821 (70%) | 1180 | 0.219 |
| Negative h-PRUNE | 256 (29%) | 625 (71%) | 881 | |

The distribution of the h-PRUNE and nm23-H1 protein expression was evaluated in a subgroup of patients (2109 breast cancer patients) on the basis of different histopathological parameters. No significant correlation was observed between h-PRUNE or nm23-H1 expression and the tumour type (ductal vs globular), pT, pN, ER and PR reactivity (table 4).

**Table 4**

| Tumour parameters | | h-prune | | | h-prune | | Total | P |
|---|---|---|---|---|---|---|---|---|
| | | Negative | Positive | | Negative | Positive | | |
| Primary tumour size | T₁ | 319 (42%) | 440 (58%) | T₁₋₂ | 765 (44%) | 985 (56%) | 1750 | 0,63 |
| | T₂ | 446 (45%) | 545 (55%) | | | | | |
| | T₃ | 54 (45%) | 65 (55%) | T₃₋₄ | 160 (45%) | 196 (55%) | 356 | |
| | T₄ | 106 (45%) | 131 (55%) | | | | | |
| Grading | G1 | 211 (40%) | 323 (60%) | G₁₋₂ | 584 (41 %) | 831 (59%) | 1415 | 0,13 |
| | G2 | 373 (42%) | 508 (58%) | | | | | |
| | G3 | 336 (48%) | 357 (52%) | G₃ | 336 (48%) | 357 (52%) | 693 | |
| Histological type | ductal | 621 (42%) | 865 (58%) | | | | 1.486 | 0,42 |
| | lobular | 117 (40%) | 174 (60%) | | | | 291 | |
| | other | 125 (49%) | 131 (51 %) | | | | 256 | |
| ER | + | 649 (42%) | 882 (58%) | | | | 1531 | 0,37 |
| | - | 204 (44%) | 259 (56%) | | | | 463 | |
| PR | + | 279 (43%) | 369 (57%) | | | | 648 | 0,34 |
| | - | 523 (42%) | 714 (58%) | | | | 1237 | |

Fluorescence *in situ* hybridization (FISH) analyses were performed on 1.016 tumours from breast cancer patients. PAC clone (279-H19) corresponding to the h-PRUNE genomic region at chromosome 1q21.3 and a control clone corresponding to the peri-centromeric region at 1 ql2 chrom o so m e were used as probes. Multiple FISH signals at 1 q21 .3 in >20% in the analysed nuclei were found in 173 (17%) cases (Table 5; Figure 9C). Considering cases with at least two gene copies for centromere and breast carcinomas with polysomy of entire chromosome 1 due to the presence of multiple centromere signals, a very low level of h-PRUNE amplification at chromosome 1q21.3 was observed (68 cases of 1.016; 6.7%). This indicates that the gene amplification mechanism occurs in a low number breast cancer cases. The increase in DNA copy number at the h-prune genomic region is significantly associated with effective statistic correlation to the presence of a positive h-prune immunostaining in tumours with trisomy (p=0.027) or tetrasomy (p=0.033) (Table 5). However, the low frequency of such a cytogenetic alteration suggests that involved alternative pathogenetic pathways determine h-PRUNE activation and increased somatic expression of h-prune in breast cancer.

**Table 5**

| Marker | No. | Disomy | % | Trisomy | % | *P* | Tetrasomy | % | *P* |
|---|---|---|---|---|---|---|---|---|---|
| h-prune negative | 440 | 386 | 88 | 54 | 12 | | 21 | 4.8 | |
| h-prune positive | 576 | 457 | 79 | 119 | 21 | *0.027* | 47 | 8.2 | *0.033* |
| Total tested | 1016 | 843 | 83 | 173 | 17 | | 68 | 6.7 | |

| | | | | | | | | | |
|---|---|---|---|---|---|---|---|---|---|
| *: multiple signals in >20% of the analysed nuclei after hybridisation with a PAC clone corresponding to the *h-prune* gene at chromosome 1q21.3; **: including also amplification of the entire chromosome 1; No: number of cases; P: Chi-squared test. | | | | | | | | | |

### Correlation between h-prune and clinicopathological parameters

To evaluate the clinicopathological role of h-PRUNE overexpression in breast cancer, immunohistochemical analyses were performed on invasive primary breast tumours using both the series of 2.109 breast cancer cases (with all of the pathological primary tumour information and up to 15-year clinical follow-up data; Table 4) and the series of 412 breast cancer cases (with a complete TNM classification and up to 10-year clinical follow-up data; Table 6).

**Table 6**

| TNM | | h-prune | | | h-prune | | Total | P |
|---|---|---|---|---|---|---|---|---|
| | | Negative | Positive | | Negative | Positive | | |
| Tumour size | T₁ | 83 (58%) | 59 (42%) | T₁₋₂ | 170 (58%) | 122 (42%) | 292 | |
| | T₂ | 87 (58%) | 63 (42%) | | | | | |
| | T₃ | 11 (44%) | 14 (56%) | T₃₋₄ | 26 (47%) | 29 (53%) | 55 | 0,099 |
| | T₄ | 15 (50%) | 15 (50%) | | | | | |
| Nodal status | N0 | 125 (71%) | 50 (29%) | N₀₋₁ | 230 (68%) | 109 (32%) | 339 | |
| | N1 | 105 (64%) | 59 (36%) | | | | | |
| | N2 | 6 (30%) | 14 (70%) | N₂₋₃ | 6 (27%) | 16 (73%) | 22 | 0.017 |
| | N3 | - | 2 (100%) | | | | | |
| Meta-stasis | M0 | 210 (67%) | 103 (33%) | | | | 313 | |
| | M1 | 14 (36%) | 25 (64%) | | | | 39 | 0.029 |

| | | | | | | | | |
|---|---|---|---|---|---|---|---|---|
| P: Chi-squared Pearson test; two tailed; 95% confidence interval; | | | | | | | | |

Tumour sections from the subset of 2,109 patients were also investigated for nm23-H1 expression by immunohistochemical analysis (Table 7).

**Table 7**

| Tumour parameter | | nm23-H1 | | | nm23-H1 | | Total | P |
|---|---|---|---|---|---|---|---|---|
| | | Negative | Positive | | Negative | Positive | | |
| Size | T₁ | 489 (68%) | 226 (32%) | T₁₋₂ | 1.162 (69%) | 517 (31 %) | 1.679 | 0,27 |
| | T₂ | 673 (70%) | 291 (30%) | | | | | |
| | T₃ | 86 (45%) | 31 (26%) | T₃₋₄ | 254 (73%) | 94 (27%) | 348 | |
| | T₄ | 168 (73%) | 63 (27%) | | | | | |
| Grading | G1 | 360 (69%) | 159 (31 %) | G₁₋₂ | 937 (68%) | 434 (32%) | 1.371 | 0.14 |
| | G2 | 577 (68%) | 275 (32%) | | | | | |
| | G3 | 501 (74%) | 179 (26%) | G₃ | 501 (74%) | 179 (26%) | 680 | |
| Histology | ductal | 1.031 (69%) | 455 (31%) | | | | 1.486 | 0.68 |
| | lobular | 201 (69%) | 90 (31 %) | | | | 291 | |
| | other | 167 (65%) | 89 (35%) | | | | 256 | |
| ER | positive | 1.076 (71%) | 447 (29%) | | | | 1.523 | 0.41 |
| | negative | 329 (70%) | 142 (30%) | | | | 471 | |
| PR | positive | 449 (69%) | 203 (31 %) | | | | 652 | 0,27 |
| | negative | 871 (71%) | 362 (29%) | | | | 1,233 | |

| | | | | | | | | |
|---|---|---|---|---|---|---|---|---|
| P: Chi-squared Pearson test; two tailed; 95% confidence interval; ER, estrogen receptor status; PR, progesterone receptor status. | | | | | | | | |

This latter subset was uninformative for the presence of distant metastases; information on nodal status was partially available for such breast cancer patients in terms of presence (N+) or absence (N-) of lymph node involvement (positive h-prune immunostaining was observed in 210/482 [44%] N+ cases and 179/496 [36%] N- cases, *P=0, 109;* analogously, no statistical correlation for nm23-H1+ immunostaining was found in these two groups). Thus, the prognostic value of each parameter was determined on the basis of the survival analysis.

On December 2002, 782 (31 %) patients were died due to disease, with the median survival of the whole being 59 months, with a follow-up of live patients of 72 months. Using Pearson's Chi-Squared test, h-prune and nm23-H1 immunostaining were evaluated for association with several pathological parameters: histological type, tumour rating (as standardised by Elston and Ellis), pT, ER, PR, and (when available) pN and M. No statistically significant correlation between either h-prune or nm23-H1 expression and tumour type (ductal vs. lobular), histological grade, pT, ER and PR reactivity were observed (Table 4). Positive h-prune immunostaining was instead significantly correlated with either the advanced nodal status (N₂-N₃ group; p=0.017) or presence of distant metastases (M₁ group; p=0.029) among the series of 412 breast cancer patients (Table 6). Statistical analysis of this series confirmed the absence of any significant association between h-prune overexpression and primary tumour size (Table 6).

Using the Cox model adjusted according to disease stage and age at diagnosis, no prognostic values of either h-prune overexpression (HR 0.61, 95% Cl 0.31-1.18, p=0.144) or nm23-H1 (RR 0.98, 95% Cl 0.90-1.07, p=0.158) were observed (Table 8 and 9; figure 10).

**Table 8**

| Statistical analysis on 2,109 breast cancer patients | | | |
|---|---|---|---|
| Marker* | Risk Ratio | 95% Cl | P |
| h-PRUNE (negative vs positive) | 0.93 | 0.78-1.09 | 0.369 |
| nm23-HI (negative vs positive) | 0.98 | 0.90-1.07 | 0.158 |
| Tumour size (pT₁ vs pT₂₋₄) | 0.81 | 0.68-0.96 | 0.017 |
| Tumour grading (G₁ vs G₂₋₃) | 0.70 | 0.59-0.83 | 0.001 |

**Table 9**

| Marker* | Risk Ratio | 95% Cl | P |
|---|---|---|---|
| h-prune (negative vs positive) | 0.61 | 0.31-1.18 | 0.144 |
| Tumour size (pT₁₋₄) | 12.49 | 4.10-38.02 | <0.001 |
| Nodal status (pN₀₋₃) | 2.43 | 1.34-4.40 | 0.003 |

Overall, tumour rating, primary tumour size, and axillary nodal status remain the parameters closely correlated to prognosis in this series of breast cancer patients (Table 8 and 9).

In conclusion the h-prune marker is therefore associated to advanced breast carcinoma status and it can be considered new marker of lymph node status in the passage from N1 to N2 status.

### EXAMPLE 4: Study on the use of IC261 that inhibits NM23 H1 and H2 phosphorylation and formation of the complex with h-prune causing an in vivo inhibition of h-prune cAMP-PDE activity.

### MATERIALS AND METHODS

Study showing that *in vitro* and *in vivo* nm23H1 and H2 proteins are phosphorylated by a Casein Kinase 1δ and phosphorylation mediates the formation of the complex (figure 11).

### Methods - in vitro

Phosphorylation *in vitro,* interaction assay and generation of phospho-nm23 antibody. Purified recombinant nm23 and mutants thereof in quantity of 100 ng were incubated in CKl standard reaction buffer as described in Garzia et al. (2003) for 1 hour at 30°C. Subsequently nm23 phosphorylated proteins were incubated with h-prune in co-IP standard buffer supplemented with 5% BSA. h-prune protein was immunoprecipitated with a polyclonal antibody, A59, against a peptide on motif III region of h-prune. The presence of different mutants of phosphorylated or not h-prune bonded nm23 was detected by subsequent Western blot analyses on immunoprecipitates, carried out with anti-His tail antibody (Qiagen) detecting at the same time h-prune and nm23 proteins.

The K73 polyclonal antibody was obtained using as immunogen a N115 to E127 phosphopeptide, phosphorylated in position S122, from nm23-H1 protein, the antiserum was used after IgG purification on the column of the resultant protein A in a non selective antibody and further affinity purification on the phosphopeptide (NIIHGSDSVKSAE) used as immunogen. This second procedure was carried out through cross-linking of 1 mg of desalted phospholipid dissolved in DMSO with the Affi-gel 25 resin (biorad) according to the manufacturer protocol. The coupled resin then was used for the affinity purification using serum of rabbit immunised with the phosphorylated and already purified and IgG peptide enriched (K73 polyclonal antibody), directly applied on Affigel bound peptide column, up to the antibody was adsorbed by the column. The column was washed for each column ml with 10 ml of 100 mM Tris-HCl, pH 8, 10 ml of 500 mM NaC, 10 mM Tris-HCl, pH 8, 10 ml of 10 mM Tris-HCl, pH 8, respectively. Elution was conducted with 0,1 M glycine, pH 3.

### METHODS-IN VIVO

### In situ protein digestion and MALDI Analysis

Trypsin, dithiotreitol and iodoacetamide were acquired from Sigma. Trifluoroacetic acid (TFA)-HPLC, 99% pure, is from Carlo Erba. All the other reagents and solvents with the highest purity were available from Baker.

Analysis was conducted on blue Coomassie stained proteins excised from SDS gel, washed twice in milliQ water gradient. Excised stains were washed firstly with CAN and then 0,1 M ammonium bicarbonate. The solution then was removed and the washing repeated twice. Protein samples were reduced by incubation in dithiotreitol (DTT) for 45 minutes at 56°C. Reducing buffer was removed by washing with ACN/AMBIC as described. Free cysteines were alkilated by incubation in 55 mM iodoacetamide for 30 minutes at room temperature in the dark. The gel particles were washed with ammonium bicarbonate and ACN. Enzymatic digestion was conducted with trypsin (12,5 ng/µl) in 10 mM ammonium bicarbonate, pH 8.0. Gel pieces were incubated at 4°C for 4 hours. Trypsin solution was then removed and a new aliquot of buffer solution was added; The samples were incubated for 18 hours at 37° C. A minimal reaction volume, enough for the complete gel rehydratation, was used. Then peptides were extracted by washing gel particles with 10mM ammonium bicarbonate and 0.1% TFA in 50% ACN at room temperature.

MALDI-TOF mass spectra were recorded using an Applied Biosystem Voyager DE-PRO instrument and a new Voyager MALDI TOF/TOF mass spectrometer. A solution mixture of peptide and alpha-cyano-hydroxy cynnamic acid (10 mg/ml in ACN/0,1% TFA in water, 2: 1, v/v) was applied to the metallic plate and dried at room temperature. Mass calibration was carried out using external standard. The first data was analysed using a software furnished by manufacturers and reported as mono-isotopic masses.

Results shown in Figure 3 demonstrate that in absence of phosphorylation, nm23H1 and nm23H2 proteins in the sites don't form the complex with h-prune.

### Cell motility study on h-prune overexpressing clones.

### Study on the inhibition by IC261.

The MDA-PRUNE #3 and MDA-PRUNE #4 cellular clone and MDA-435 C-100 cells (control cells) were treated with 8 µM dipyridamole or 50 µM IC261 (Casein Kinase Iδ and ε inhibitor; Calbiochem, Nottingham, UK) for 24 hours or 8 hours, respectively. Cellular motility assays then were applied to "boiden and chamber" cells using 0,5% Fetal Serum FCS concentration as chemo-attractant. Then cells were counted under microscope and a statistic analysis for achieved data was applied (Figure 13A).

Cells were counted under microscope, cell motility values are arithmetic means ± SD for five independent assays, every one performed in duplicate (MDA-clones prune #3 and #4/MDA-clones prune #3 and #4 with IC261 p < 0,002).

### In vitro cellular motility assay

Stable h-prune overexpressing MDA clones were achieved as described in D'Angelo et al 2004. The control MDA-C100 breast cancer cell line was used in the cell motility assay (Leone et al. 1993a; Leone et al. 1993b). Cellular motility was determined using the trans-well technology (6 well, Corning-Costar) using 0.5% FCS, final concentrations as chemo-attractants (see D'Angelo et al. 2004). *In vitro* h-prune inhibition motility assay was carried out as follows. MDA-prune (clones #3 and #4) and MDA-C100 were incubated with dipyridamole (8 µM, a 10-fold higher concentration with respect to its IC₅₀) for 24 h to obtain the complete enzyme inactivation, the same cellular lines were incubated with IC261 at a concentration of 50 µM for 8h, in which condition the highest reduction of nm23 phosphorylation without signal of cellular death can be observed, and then the motility assay was repeated as described above. All results are the average of five independent experiments, every one conducted in duplicate. Statistical analysis was performed with the T-test method deteriorated available in the site http://www.graphpad.com/quickcaics/index.cfm, values with a P≤0,05 are considered statistically significant.

### Study on the total content of cAMP-PDE in h-prune overexpressing cells and subjected to IC261 administration.

MDA-Prune#4 cells were as such or treated with 50 µM IC261 for 8 hours. Then cells were lysed and tested for their cAMP-PDE total content using the Amersham "scintillation proximity assay" method. In presence of IC261 no alteration of the cAMP phosphodiesterase activity of recombinant h-prune (Figure 13 B, right) was detected whereas it was pointed out the ability to sensitively lower total content of cAMP-PDE activity in prune overexpressing MDA prune #4 clone, showing that the h-prune and nm23 complex is responsible for cell PDE-cAMP increase (figure 13B, left) and that such complex can be inhibited by IC261 *in vivo.*

### A. PDE activity assay vs cAMP

PDE activity was measured with a scintillation proximity assay (Amersham-Pharmacia Biotech). Samples, 20 µg of MDA-prune#4 total extract and MDA-C100 extract used as control, were incubated at 30°C in 100 µl of assay buffer (50 mM Tris-HCl [PH 7.4], 8.3 mM MgCl₂, 1.7 mM EGTA) containing desired cAMP concentrations as substrate (unlabelled vs 3H-labelled ratio 3:1). All reactions, including blanks with alone buffer, were carried out in triplicate and allowed to proceed for an incubation time giving a substrate turnover (empirically determined) < 25%. The reactions were quenched by adding 50 µl of Yttrium silicate SPA pearls (Amersham). Enzymatic activities for discovered radio-labelled product quantity were calculated according to the manufacturer protocol. The treatment with IC261 was the same as for motility assay described above.

### RESULTS

The obtained results show that by inhibiting the Casein Kinase 1 δ using IC261 it is possible to inhibit nm23-H1 and H2 phosphorylation and therefore the bonding with h-prune protein *in vivo.* By using this drug it is possible therefore to inhibit the formation of the h-prune-nm23 complex, being this last dependent on phosphorylations in 8120, 8122, 8125 region (NIIHGSDSVESAEKE) by Casein Kinase I. This study allows to use IC261 for inhibiting the binding of nm23H1 and H2 to h-prune and inhibiting cellular motility induced by the protein complex.

A new procedure can foresee the use of new peptides permeable in the cell and competitive for the binding of h-prune, poaching quota of in vivo phosphorylated nm23-H1 and H2, whose sequence derives from the amino acidic sequence of nm23-H1 and H2 range of 8120, 8122 and 8125 serines (example H1: NIIHGSDSVESAEKE followed by the permeable peptide sequence of the region of the HIV TAT protein; GGGYGRKKRRQRRR; 95% of purity synthesized by PRIMM). Such or similar peptides are able to compete with Casein Kinasi I phosphorylation *in vivo,* on the H1 and H2 wild type proteins and reduce quota of phosphorylated nm23 responsible for h-prune complex formation and increase of h-prune cAMP-PDE activity in the cell and finally induction of the cellular motility. Also a control peptide with recognition sequence of the Casein Kinasi I cramble referred as (H1-: SDEIGKVSENIAHSE followed by permeable peptide sequence GGGYGRKKRRQRRR) was synthesised. Using this technology it is possible to target more specifically the inhibition of interaction between h-prune and nm23 *in vivo.*

### EXAMPLE 5: Preparation of monoclonal antibody against h-prune (4G3/4, deposited at the CBA in Genoa on 10/12/2004).

h-prune protein, fused at N terminal a tail and produced in *E*. *Coli* through a pMaltose vector, was gel purified. The mice were immunized for 5 weeks (100 ug protein per injection) and the spleen was taken for preparing some hybridoma cultures. It was necessary approximately 2 mg of protein antigen for immunisation and analysis. Primm can provide conjugated synthetic peptides for the immunization in the case the protein wasn't available. The development and production of monoclonal antibodies are set out in different phases:
- peptide synthesis and peptide conjugation (where necessary).
- synthesis of about 10 mg of peptide, purity > 70%
- conjugation of 3-4 mg of peptide to a carrier protein (as antigen)
- conjugation of 1-2 mg of peptide to a different carrier protein for ELISA screening.

### Phase I (1,5 months):

Immunization of 3 BALB/c mice with antigen. Procedure for the quantitative titration of specific immunoglobulins in the immunised animal sera. ELISA assay of the immune response and animal selection for the following fusion of spleen cells.

### Phase II (2 months)

Splenectomy and fusion of the splenocytes with myelosis cells; hybridoma selection through ELISA, freezing of the most positive clones.

### Phase III (1-1,5 months)

Cloning and subcloning of positive clones (1/2 or 4) through limiting dilution. Freezing of hybridomas (2 aliquot/clone). Viability, productivity and stability assays after unfreezing of an aliquot.

### Phase IV (1-1,5 months).

Production and purification of 0,5-4 mg of any mAb in cellular culture from 0,5 I of supernatant.

Selected hybridoma produces murine IgMs which were purified from hybridoma culture supernatant. The mAb quantity employed for immunocytochemistry is equal to a mAb solution diluted 1: 100 to 300 ug/ml. The antibody works and recognises both human and murine prunes. The detection of endogenous protein is carried out using ICC, IF and Western blot (Figure 14).

The antibody purification was carried out according to the following IgM purification protocol: supernatant of the lysed cells was subjected to a flow of 1 ml/min. The HiTrap IgM was equilibrated (1 ml, Amersham-Pharmacia) with 5 volumes of 20mM sodium phosphate, pH 7,4, 0,8 M (NH₄) 2S0₄ (Buffer A); 5 volumes of 20mM sodium phosphate pH 7,4 (Buffer B); 5 volumes of 20mM sodium phosphate pH 7,4, 30% isopropanol (Buffer C). ELISA assays were conducted against peptide and purified whole protein for determining the specificity of produced antigen and clone selection.

Introduce hybridoma supernatant in the column and repeat thrice the elution process with the three buffers, so set out: column washing with 15 volumes of Buffer A (15 ml); column elution with 12 volumes (12 ml) of Buffer B (1 ml fraction collection); reading of the fraction absorbance at 280 nm and collection of the fractions with absorbency < 0,1 O.D.; fraction dialysis with tubes with a cut-off between 12000-14000 against a 1X PBS buffer overnight at 4°C. To regenerate the column use 7 ml of buffer C. Column storage in buffer B + 20% ethanol.

### BIBLIOGRAPHY

- Aravind, I. and Koonin, E.V. (1998). Trends in biochemical sciences 23,17-19.
- Timmons, L. and Shearn, A., (1996). Genetics 144, 1589-1600.
- Lombardi, D., Lacombe, M.L. and Paggi, M.G., (2000). J. Cell Physiol 182, 144-149.
- Keen, J.C., Davidson, N.E., (2003). Cancer 97, 825-833.
- Domchek, S.M., Weber, B.L, (2002). Curr Opin Oncol 14, 589-593.
- Kuru, B., Camlibel, M., Ali Gulcelik, M., Alagol, H., (2003). J.Surg. Oncol 83, 167-172.
- Morabito, A., Magnani, E., Gion, M., (2003). Clin Breast Cancer 3, 381-390.
- Florenes, V.A, Aamdal, S., Myklebost, O., Maelandsmo, G.M. Bruland, O.S. and Fodstad, O., (1992). Cancer Res 52, 6088-6091.
- Steeg, P.S., (2003). Nat Rev Cancer 3, 55-63.
- Frejie, J.M., Blay, P., Macdonald, N.J., Manrow, King., and Steeg, P.S., (1997). J Biol Chem 272, 5525-5532.
- Hartsough, M.T., Clare, S.E., Mair, M., Elkaloun, AG., Sgroi, D., Osborne, C.K, Clarke, G. and Steeg, P.S. (2001). Cancer Res 61, 2320-2327.
- Frejie, J.M., Lawrence J.A., Hollingshead M.G., De la Rosa, A., Narayanan, V., Grever, M., Sausville, E.A, Paull, K, and Steeg P.S. (1997). Nat Med 3, 395-401.
- Kantor, J.O., Mc Cormick, B., Steeg, P.S., and Zetter, B.R., (1993) Cancer Res 53, 1971-1973.
- Leone A, Flatow, U., Van Houtte K, and Steeg P.S. (1993). Oncogene 8, 2325-2333.
- Howlett, A.R., Petersen, O.W., Steeg, P.S. and Bissell M.J. (1994) J Natl Cancer Inst 86, 1838-1844.
- Hartsough, M.T., and Steeg P.S., (1998). Am J Hum Genet 63,6 10.
- Lombardi D., L.M.L., Pagggi M.G., (2000) J. Cell Physiol. 182, 144149.
- Hartsough M.T. and Steeg P.S. (2000). J. Bioenerg Biomembr 32, 301-308.
- Mao, H., Liu, H., Fu, X., Fang, Z., Abrams, J., and Worsham, M.J. (2001). Int J Oncol 18, 587-591.
- Tseng Y. H., Vicent, D., Zhu, J., Niu, Y., Adeyinka, A., Moyers, J.S., Watson, P.H., and Kahn, C.R (2001). Cancer Res 61, 2071-.2079.
- Reymond A, Valorio, S., Merla, G., Al-Maghteh, M., Zuffardi, O., Bulfone A, Ballabio A, and Zollo, M., (1999). Oncogene 18, 7244 7252.
- Forus A, D'Angelo A., Henriksen, J., Merla, G., Maelandsmo, G.M. Florenes, Y.A, Olivieri S., Bjerkehagen, B., Meza-Zepeda, L.A., Del Vecchio Blanco, F., Muller, C., Sanvito F., Kononen, J., Nesland, J.M. Fodstad O., Reymond A, Kallioniemi, O.P., Arrigoni G., Ballabio A, Myklebost, O., and Zollo M., (2001). Oncogene 20, 6881-6890.
- Altshul, S.F., Madden, T.L., Schaffer A.A., Zhang Z., Miller W., and Lipman, D.J. (1997). Nucleic Acids Research 25, 3389-3402.
- Notredame C., Higgins D.G., and Heringa J., (2000). J Mol Biol 302 205-217.
- Macdonald, N.J., Freije, J.m.p, Stracke, M.L, Manrow R.E., and Steeg, P.S. (1996). J. Biol Chem 271, 25107-25116.
- Garzia, L, Andrè A., Amoresano. A., D'Angelo, A., Martusciello R, Cirulli, C., Turumi, T, Marino G., and Zollo M., (2003). Biotechniques vol 35, 384-391.
- Fisher D.A, Smith, J.F., Pillar, J.S., St Denis, S.H. and Cheng, J.B. (1998). J Biol Chem 273, 15559-15564.
- Leone, A., Seeger R.C., Hong C.C.M., Hu, Y.Y., Arboleda M.J. Brodeur G.M., Stram, D., Slamon D.J., and Steeg, P.S. (1993). Oncogene 8, 855-865.
- Yamagata, A., Kakuta, Y., Masui, R and Fukuyama, K (2002). Proc Natl Acad Sci Usa 99, 5908-5912.
- Ahn, S., Milner, A. J. Futterer, K, Konopka, M., Ilias, M., Young T.W. and White S,A (2001). J. Mol Biol 313, 797, 811.
- Aravind L, and Koonin EV. (1998). Trends Biochem Sci 23, 469 472.
- Aravind L., and Koonin EV. (1998). Nucleic Acids Res 26, 3746 3752.
- Galperin M.Y., Natale D.A, Aravind L, and Koonin E.V. (1999). J. Mol Microbiol Biotechnol 1, 303-305.
- Aravind L., (1999). In Silico Biol 1, 69-91.
- Hejna, M.; Raderer M., and Zielinsky, C.C. (1999). J Natl Cancer Inst 91, 22-36.
- Stteg, P.S., De la Rosa, A., Flatow, U., Macdonald N.J., Benedictc M., and Leone A., (1993). Breast Cancer Res Treat 25, 175-187.
- Wagner, P.D., Steeg, P.S. and Vu, N.D. (1997). Proc Natl A-cad Sci USA 94, 9000-9005.
- Simon, R, Nocito, A., Hubscher, T., (2001). J. Natl Cancer Inst, 93,1141-1146.
- Simon, R, Sauter, G., (2002). Exp Hematol, 30(12), 1365-1372.
- D'Angelo, A., Garzia, L, Andrè, A., (2003). Cancer Cell 2003.
- Muresu, R, Sini, M. C., Cossu, Á., (2002). Eur J Cancer 38, 1802 1809.

## Claims

1. Peptide comprising the following amino acidic sequence: NIIHGSDSVESAEKEGGGYGRKKRRQRRR (SEQ ID No 10) **characterised in that** it is permeable.

2. Peptide comprising the following amino acidic sequence: NIIHGSDSVESAEKE GGGYGRKKRRQRRR (SEQ ID No 10) and **characterised in that** it is permeable and it is an inhibitor of h-Prune cyclic nucleotide phosphodiesterase activity, for use in medical field.

3. Use of the peptide according to claim 1 for the preparation of a medicament for prevention and treatment of tumour metastases **characterised by** an overexpression of h-PRUNE.

4. Use according to claim 3, wherein tumours **characterised by** an overexpression of h-PRUNE are breast carcinoma, sarcoma, neuroblastoma, prostate tumour, pancreatic tumour, colon carcinoma tumour, rectal tumour, medulloblastoma, epitelioma, epatocarcinoma, cell T or cell B lymphomas, myeloma and melanoma, and pulmonary tumour.

5. Screening method for h-PRUNE-inhibiting compounds, comprising the following phases:
a) selection of at least a phosphoesterase (PDE) inhibiting compound or derivative, structural analogue or isomer thereof;
b) administration of said at least one compound at concentration between 0,05 µM and 10 µM in a cell line overexpressing h-PRUNE, wherein said cellular line is MDA-C100 435 prune #4;
c) quantitative analysis of the cyclic nucleotide phosphodiesterase activity of h-PRUNE and/or analysis of cellular motility versus concentration of said at least one compound and chemo-attractant and selection of compound able to inhibit said phosphodiesterase activity between the values from 0.01 to 1 pmol/min⁻¹/ug⁻¹ and/or inhibit said motility up to the attainment of the values between 200 and 1200 cells.

6. Screening method according to claim 5, wherein the quantitative analysis of step c) is carried out by hydrolysis tests of the c-AMP and/or c-GMP substrate.

7. Screening method according to claim 6, wherein the substrate is used at concentration between 0,008 µM and 1 µM.

8. Method for in vitro detection of h-PRUNE in a biological sample for metastases diagnosis of tumours **characterised by** an h-PRUNE overexpression by immunological assay comprising the following steps:
a) bring into contact said biological sample with the anti-h-PRUNE monoclonal antibody able to recognise and bind selectively the h-PRUNE recombinant protein, **characterised in that** it belongs to the IgM immunoglobulin class and is produced by 4G3/4 clone (deposited at the CBA in Genoa on 10/12/2004);
b) detection of the antigen-antibody complex;
c) quantitative analysis of the antigen-antibody complex.

9. Method according to claim 8, wherein said biological sample is a tissue section or biological fluid.

10. Method according to any one of claims 8-9, wherein said anti-h-PRUNE antibody is labelled with a radioisotope, fluorescent molecule or enzyme.

11. Method for in vitro detection of h-PRUNE according to claim 8, wherein said detection and quantitative analysis of the antigen-antibody complex are performed by immunohistochemistry, immunoprecipitation, immunofluorescence, ELISA, immunoblotting analyses.

12. Diagnostic kit for the detection of h-PRUNE in a biological sample for metastases diagnosis of tumours **characterised by** an h-PRUNE overexpression comprising the anti-h-PRUNE monoclonal antibody that belongs to the IgM immunoglobulin class and is produced by 4G3/4 clone (deposited at the CBA in Genoa on 10/12/2004).

13. Diagnostic kit according to claim 12, wherein the tumours **characterised by** an h-PRUNE overexpression are breast carcinoma, sarcoma, neuroblastoma, melanoma.

14. Diagnostic kit according to claim 12, wherein said anti-h-PRUNE monoclonal antibody is labelled with a radioisotope, fluorescent molecule or enzyme.

15. Monoclonal murine antibody able to recognise and bind selectively the h-PRUNE recombinant protein, **characterised in that** it belongs to the IgM immunoglobulin class and is produced by 4G3/4 clone (deposited at the CBA in Genoa on 10/12/2004).

## Patentansprüche

1. Peptid, umfassend die folgende Aminosäuresequenz: NIIHGSDSVESAEKEGGGYGRKKRRQRRR (SEQ ID Nr. 10), welches **dadurch gekennzeichnet ist, dass** es permeabel ist.

2. Peptid, umfassend die folgende Aminosäuresequenz: NIIHGSDSVESAEKEGGGYGRKKRRQRRR (SEQ ID Nr. 10) und **dadurch gekennzeichnet, dass** es permeabel und ein Inhibitor der cyclisches Nukleotid-Phosphodiesterase-Aktivität von h-Prune ist, zur Verwendung auf dem Gebiet der Medizin.

3. Verwendung des Peptids nach Anspruch 1 zur Herstellung eines Medikaments zur Verhütung und Behandlung von Tumormetastasen, die durch eine Überexpression von h-PRUNE gekennzeichnet sind.

4. Verwendung nach Anspruch 3, wobei die Tumore, die durch eine Überexpression von h-PRUNE gekennzeichnet sind, Brustkarzinom, Sarkom, Neuroblastom, Prostatatumor, Pankreastumor, Dickdarmkarzinom, Rektaltumor, Medulloblastom, Epitheliom, Hepatokarzinom, T-Zell- oder B-Zell-Lymphome, Myelom und Melanom sowie Lungentumor sind.

5. Screeningverfahren für h-PRUNE-inhibierende Verbindungen, umfassend die folgenden Phasen:
a) Selektion von mindestens einer Phosphoesterase (PDE)-inhibierenden Verbindung oder eines Derivats, strukturellen Analogons oder Isomers davon;
b) Verabreichung der mindestens einen Verbindung in einer Konzentration zwischen 0,05 µM und 10 µM an eine Zelllinie, die h-PRUNE überexprimiert, wobei die Zelllinie MDA-C100 435 prune #4 ist;
c) quantitative Analyse der cyclisches Nukleotid-Phosphodiesterase-Aktivität von h-PRUNE und/oder Analyse der Zellbeweglichkeit gegen die Konzentration der mindestens einen Verbindung und eines Chemo-Attraktors und Auswahl einer Verbindung, welche die Phosphodiesterase-Aktivität auf Werte zwischen 0,01 bis 1 pmol/min⁻¹/ug⁻¹ inhibieren und/oder die Beweglichkeit bis zum Erhalt von Werten zwischen 200 und 1200 Zellen inhibieren kann.

6. Screeningverfahren nach Anspruch 5, wobei die quantitative Analyse von Schritt c) durch Hydrolysetests des c-AMP-und/oder des c-GMP-Substrats durchgeführt wird.

7. Screeningverfahren nach Anspruch 6, wobei das Substrat in einer Konzentration zwischen 0,008 µM und 1 µM verwendet wird.

8. Verfahren zum in Vitro-Nachweis von h-PRUNE in einer biologischen Probe für die Metastasen-Diagnose von Tumoren, die durch eine h-PRUNE-Überexpression **gekennzeichnet sind, durch** einen immunologischen Assay, umfassend die folgenden Schritte:
a) Kontaktieren der biologischen Probe mit einem monoklonalen Anti-h-PRUNE-Antikörper, welcher in der Lage ist, rekombinantes h-PRUNE-Protein zu erkennen und selektiv zu binden, und welcher **dadurch** gekennzeichnet ist, dass er zur Immunoglobulinklasse IgM gehört und von dem Klon 4G3/4 (hinterlegt bei der CBA in Genua am 10. Dezember 2004) erzeugt wird;
b) Nachweis des Antigen-Antikörper-Komplexes;
c) quantitative Analyse des Antigen-Antikörper-Komplexes.

9. Verfahren nach Anspruch 8, wobei die biologische Probe eine Gewebesektion oder ein biologisches Fluid ist.

10. Verfahren nach irgend einem der Ansprüche 8 bis 9, wobei der Anti-h-PRUNE-Antikörper mit einem Radioisotop, einem fluoreszierenden Molekül oder einem Enzym markiert ist.

11. Verfahren zum in vitro-Nachweis von h-PRUNE gemäß Anspruch 8, wobei der Nachweis und die quantitative Analyse des Antigen-Antikörper-Komplexes durch Immunhistochemie, Immunpräzipitation, Immunfluoreszenz, ELISA oder Immunoblot-Analysen durchgeführt wird.

12. Diagnosekit zum Nachweis von h-PRUNE in einer biologischen Probe zur Metastasen-Diagnose von Tumoren, die durch eine h-PRUNE-Überexpression gekennzeichnet sind, umfassend den monoklonalen Anti-h-PRUNE-Antikörper, der zur Immunglobulinklasse IgM gehört und von dem Klon 4G3/4 (hinterlegt bei der CBA in Genua am 10. Dezember 2004) erzeugt wird.

13. Diagnosekit nach Anspruch 12, wobei die durch eine h-PRUNE-Überexpression gekennzeichneten Tumore Brustkarzinom, Sarkom, Neuroblastom und Melanom sind.

14. Diagnosekit nach Anspruch 12, wobei der monoklonale Anti-h-PRUNE-Antikörper mit einem Radioisotop, einem fluoreszierenden Molekül oder einem Enzym markiert ist.

15. Monoklonaler Maus-Antikörper, welcher zur Erkennung und selektiven Bindung des rekombinanten h-PRUNE-Proteins in der Lage ist, **dadurch gekennzeichnet, dass** er zur Immunglobulinklasse IgM gehört und von dem Klon 4G3/4 (hinterlegt bei der CBA in Genua am 10. Dezember 2004) erzeugt wird.

## Revendications

1. Peptide comprenant la séquence d'acides aminés suivante : NIIHGSDSVESAEKEGGGYGRKKRRQRRR (ID seq. n° 10), **caractérisé en ce qu'**il est perméable.

2. Peptide comprenant la séquence d'acides aminés suivante : NIIHGSDSVESAEKEGGGYGRKKRRQRRR (ID seq. n° 10) et **caractérisé en ce qu'**il est perméable et qu'il est un inhibiteur de l'activité phosphodiestérase du nucléotide cyclique h-PRUNE, à utiliser dans le domaine médical.

3. Utilisation du peptide selon la revendication 1, pour la préparation d'un médicament destiné à la prévention et au traitement de métastases tumorales **caractérisées par** une surexpression de h-PRUNE.

4. Utilisation selon la revendication 3, dans laquelle les tumeurs **caractérisées par** une surexpression de h-PRUNE sont le carcinome du sein, le sarcome, le neuroblastome, la tumeur de la prostate, la tumeur pancréatique, la tumeur du carcinome du côlon, la tumeur rectale, le médulloblastome, l'épithéliome, l'épatocarcinome, les lymphomes des cellules T ou des cellules B, le myélome et le mélanome, et la tumeur pulmonaire.

5. Procédé de sélection de composés inhibiteurs de h-PRUNE, comprenant les étapes suivantes :
a) sélection d'au moins un composé inhibant une phosphoestérase (PDE) ou d'un dérivé, analogue structurel ou isomère de celui-ci ;
b) administration dudit composé, au moins au nombre de un, dans une concentration entre 0,05 µM et 10 µM dans une lignée cellulaire surexprimant h-PRUNE, où ladite lignée cellulaire est MDA-C100 435 prune #4 ;
c) analyse quantitative de l'activité phosphodiestérase de nucléotide cyclique de h-PRUNE et/ou analyse de la motilité cellulaire en fonction de la concentration dudit composé et facteur chimiotactique, au moins au nombre de un, et sélection d'un composé capable d'inhiber ladite activité phosphodiestérase entre les valeurs de 0,01 à 1 pmol/min⁻¹/ug⁻¹ et/ou d'inhiber ladite motilité jusqu'à atteindre les valeurs entre 200 et 1200 cellules.

6. Procédé de sélection selon la revendication 5, dans lequel l'analyse quantitative de l'étape c) est effectuée par des tests d'hydrolyse du substrat c-AMP et/ou c-GMP.

7. Procédé de sélection selon la revendication 6, dans lequel le substrat est utilisé dans une concentration comprise entre 0,008 µM et 1 µM.

8. Procédé de détection in vitro de h-PRUNE dans un échantillon biologique pour le diagnostic de métastases de tumeurs **caractérisées par** une surexpression de h-PRUNE par dosage immunologique, comprenant les étapes suivantes :
a) mise en contact dudit échantillon biologique avec l'anticorps monoclonal anti-h-PRUNE capable de reconnaître et de lier sélectivement la protéine recombinante h-PRUNE, **caractérisée en ce qu'**il appartient à la classe des immunoglobulines IgM et est produit par le clone 4G3/4 (déposé au CBA de Genève le 10/12/2004) ;
b) détection du complexe antigène-anticorps ;
c) analyse quantitative du complexe antigène-anticorps.

9. Procédé selon la revendication 8, dans lequel ledit échantillon biologique est une section de tissu ou un fluide biologique.

10. Procédé selon l'une quelconque des revendications 8 à 9, dans lequel ledit anticorps anti-h-PRUNE est marqué avec un radio-isotope, une molécule fluorescente ou une enzyme.

11. Procédé de détection in vitro de h-PRUNE selon la revendication 8, dans lequel lesdites détection et analyse quantitative du complexe antigène-anticorps sont réalisées par des analyses d'immunohistochimie, d'immunoprécipitation, d'immunofluorescence, ELISA ou d'immunoempreinte.

12. Kit de diagnostic pour la détection de h-PRUNE dans un échantillon biologique pour diagnostiquer des métastases de tumeurs **caractérisées par** une surexpression de h-PRUNE comprenant l'anticorps monoclonal anti-h-PRUNE qui appartient à la classe des immunoglobulines IgM et est produit par le clone 4G3/4 (déposé au CBA de Genève le 10/12/2004).

13. Kit de diagnostic selon la revendication 12, dans lequel les tumeurs **caractérisées par** une surexpression de h-PRUNE sont le carcinome du sein, le sarcome, le neuroblastome et le mélanome.

14. Kit de diagnostic selon la revendication 12, dans lequel ledit anticorps monoclonal anti-h-PRUNE est marqué avec un radio-isotope, une molécule fluorescente ou une enzyme.

15. Anticorps monoclonal murin capable de reconnaître et de lier sélectivement la protéine recombinante h-PRUNE, **caractérisé en ce qu'**il appartient à la classe des immunoglobulines IgM et est produit par le clone 4G3/4 (déposé au CBA de Genève le 10/12/2004).


### SEQUENCE LISTING

<110> zollo, Massimo
<120> Use of enzymatic inhibitors of h-PRUNE for the prevention and treatment of the metastases of tumours overexpressing h-PRUNE
<130> PCT25791
<150> RM2003 000572
   <151> 2003-12-11
<160> 12
<170> PatentIn version 3.2
<210> 1
   <211> 22
   <212> DNA
   <213> Artificial
<220>
   <223> Primer forward h-PRUNE
<400> 1
   agagatcttg gacaggcaaa ct 22
<210> 2
   <211> 22
   <212> DNA
   <213> Artificial
<220> .
   <223> Primer Reverse h-PRUNE
<400> 2
   ccatgttgac acagtccagg at 22
<210> 3
   <211> 15
   <212> DNA
   <213> Artificial
<220>
   <223> Probe h-PRUNE 6FAM
<400> 3
   ctgcatggaa ccatc 15
<210> 4
   <211> 18
   <212> PRT
   <213> Artificial
<220>
   <223> Antigen for the monoclonal antibody anti h-PRUNE
<400> 4
   Ala Leu Glu Glu Ala Val Ala Glu Val Leu Asp His Arg Pro Ile Glu
   1               5                   10                  15
   Pro Lys
<210> 5
   <211> 42
   <212> DNA
   <213> Artificial
<220>
   <223> Primer h-PRUNE delta
<400> 5
   gtagcagagg tgctagccgc tgcagccatc gagccgaaac ac 42
<210> 6
   <211> 21
   <212> DNA
   <213> Artificial
<220>
   <223> Primer D28A h-PRUNE
<400> 6
   gaagcctgtg ctttggactc c 21
<210> 7
   <211> 33
   <212> DNA
   <213> Artificial
<220>
   <223> Primer 106A h-PRUNE
<400> 7
   accctcatcc ttgtcgctca tcatatctta tcc 33
<210> 8
   <211> 30
   <212> DNA
   <213> Artificial
<220>
   <223> Primer D179a h-PRUNE
<400> 8
   gaaccatcat cctggcatgt gtcaacatgg 30
<210> 9
   <211> 15
   <212> PRT
   <213> Artificial
<220>
   <223> Inhibitor of h-prune H1
<400> 9
   Asn Ile Ile His Gly Ser Asp Ser Val Glu Ser Ala Glu Lys Glu
   1               5                   10                  15
<210> 10
   <211> 29
   <212> PRT
   <213> Artificial
<220>
   <223> Permeable inhibitor of h-prune H1
<400> 10
   Asn Ile Ile His Gly Ser Asp Ser Val Glu Ser Ala Glu Lys Glu Gly
   1               5                   10                  15
   Gly Gly Tyr Gly Arg Lys Lys Arg Arg Gln Arg Arg Arg
               20                  25
<210> 11
   <211> 167
   <212> PRT
   <213> Artificial
<220>
   <223> h-prune sequence
<400> 11
   Met Tyr Asp Val Pro Asp Tyr Ala Ser Leu Gly Ser Pro Val Glu Met
   1 5 10 15
   Ala Asn Leu Glu Arg Thr Phe Ile Ala Ile Lys Pro Asp Gly Val Gln
   20 25 30
   Arg Gly Leu Val Gly Glu Ile Ile Lys Arg Phe Glu Gln Lys Gly Phe
   35 40 45
   Arg Leu Val Ala Met Lys Phe Leu Arg Ala Ser Glu Glu His Leu Lys
   50 55 60
   Gln His Tyr Ile Asp Leu Lys Asp Arg Pro Phe Phe Pro Gly Leu Val
   65 70 75 80
   Lys Tyr Met Asn Ser Gly Pro Val Val Ala Met Val Trp Glu Gly Leu
   85 90 95
   Asn Val Val Lys Thr Gly Arg Val Met Leu Gly Glu Thr Asn Pro Ala
   100 105 110
   Asp Ser Lys Pro Gly Thr Ile Arg Gly Asp Phe Cys Ile Gln Val Gly
   115 120 125
   Arg Asn Ile Ile His Gly Ser Asp Ser Val Lys Ser Ala Glu Lys Glu
   130 135 140
   Ile Ser Leu Trp Phe Lys Pro Glu Glu Leu Val Asp Tyr Lys Ser Cys
   145 150 155 160
   Ala His Asp Trp Val Tyr Glu
   165
<210> 12
   <211> 15
   <212> PRT
   <213> Artificial
<220>
   <223> control peptide H1 (-) Casein kinase I
<400> 12
   Ser Asp Glu Ile Gly Lys Val Ser Glu Asn Ile Ala His Ser Glu
   1 5 10 15